(19) Europäisches Patentamt
European Patent Office
Office européen des brevets

(11) Veröffentlichungsnummer: **0 056 089**
**B1**

# (12) EUROPÄISCHE PATENTSCHRIFT

(45) Veröffentlichungstag der Patentschrift: **07.03.84**

(51) Int. Cl.³: **C 07 D 405/06**, A 01 N 43/64, A 01 N 43/50

(21) Anmeldenummer: **81108835.0**

(22) Anmeldetag: **24.10.81**

## (54) Azolylalkyl-2,3-dihydrobenzofurane, diese enthaltende Fungizide und Verfahren zu ihrer Herstellung.

(30) Priorität: **31.12.80 DE 3049542**

(43) Veröffentlichungstag der Anmeldung: **21.07.82 Patentblatt 82/29**

(45) Bekanntmachung des Hinweises auf die Patenterteilung: **07.03.84 Patentblatt 84/10**

(84) Benannte Vertragsstaaten: **AT BE CH DE FR GB IT LI LU NL SE**

(56) Entgegenhaltungen:
**DE - A - 2 063 857**
**DE - A - 2 203 373**
**DE - A - 2 638 470**

(73) Patentinhaber: **BASF Aktiengesellschaft, Carl-Bosch-Strasse 38, D-6700 Ludwigshafen (DE)**

(72) Erfinder: **Rentzea, Costin, Dr., Neuenheimer Landstrasse 72, D-6900 Heidelberg (DE)**
Erfinder: **Feuerherd, Karl-Heinz, Dr., Berner Weg 34, D-6700 Ludwigshafen (DE)**
Erfinder: **Zeeh, Bernd, Dr., Thorwaldsenstrasse 5, D-6700 Ludwigshafen (DE)**
Erfinder: **Pommer, Ernst-Heinrich, Dr., Berliner Platz 7, D-6703 Limburgerhof (DE)**

**EP 0 056 089 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Azolylalkyl-2,3-dihydrobenzofurane, diese enthaltende Fungizide und Verfahren zu ihrer Herstellung

Die vorliegende Erfindung betrifft Azolylalkyl-2,3-dihydrobenzofurane, Verfahren zu ihrer Herstellung, fungizide Mittel, die diese Verbindungen enthalten, Verfahren zur Bekämpfung von phytopathogenen Pilzen mit diesen Substanzen sowie die Herstellung fungizider Mittel mit diesen Substanzen.

Es ist bekannt, dass Imidazolderivate wie beispielsweise das 1-(2-(2',4'-Dichlorphenyl)-2-(2'-propenyl-1'-oxy)-ethyl)-1H-imidazol (DE-OS 2063857) und Triazolderivate, z.B. das 2,2-Dimethyl-4-(1,2,4-triazol-1-yl)-5-phenylpentan-3-on (DE-OS 2638470) eine gute fungizide Wirksamkeit zeigen. Die Wirkung ist jedoch bei niedrigen Aufwandmengen nicht immer ausreichend. Ferner ist die fungitoxische Wirkung mit einer hohen Phytotoxizität verbunden, so dass bei den für die Bekämpfung von phytopathogenen Pilzen, beispielsweise bei der Bekämpfung von Mehltau oder Rostpilzen notwendigen Konzentrationen auch wichtige Kulturpflanzen geschädigt oder in ihrem Wachstum stark gehemmt werden. Aus diesen Gründen sind sie für den Gebrauch als Mittel zur Bekämpfung von phytopathogenen Pilzen nicht immer und nicht bei allen Pflanzenarten geeignet.

Es wurden nun Verbindungen gefunden, die eine sehr gute fungizide Wirksamkeit bei ausgezeichneter Pflanzenverträglichkeit besitzen.

Gegenstand der Erfindung sind Azolylalkyl-2,3-dihydrobenzofurane der Formel I

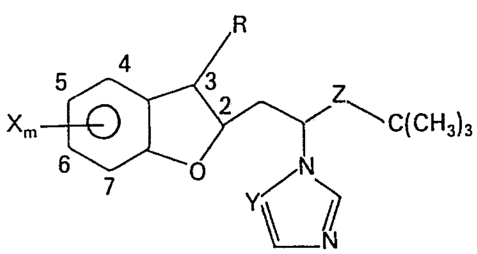

(I),

in der

R Wasserstoff oder Alkyl mit 1 bis 4 C-Atomen bedeutet,

X Wasserstoff, Fluor, Chlor, Brom, Jod, Nitro, Trifluormethyl, Alkyl mit 1 bis 5 C-Atomen, Alkoxy mit 1 bis 3 C-Atomen oder Alkenyl mit 2 bis 3 C-Atomen, Phenyl oder Phenoxy bedeutet und

m eine ganze Zahl von 1 bis 4 bedeutet, wobei die einzelnen Gruppen X gleich oder verschieden sind, wenn m grösser als 1 ist,

Y N oder CH bedeutet und

Z eine -CO- oder $-CR^1OR^2$-Gruppe bedeutet, in welcher

$R^1$ Wasserstoff oder $C_1$–$C_4$-Alkyl bedeutet und

$R^2$ Wasserstoff, einen gegebenenfalls durch Chlor substituierten Alkylrest mit 1 bis 6 C-Atomen oder einen gegebenenfalls durch Chlor substituierten Alkenylrest mit 3 bis 5 C-Atomen oder einen Alkinylrest mit 3 bis 4 C-Atomen oder Benzyl bedeutet, wobei der Benzylrest durch Fluor, Chlor, Brom, Nitro, Trifluormethyl und Alkyl mit 1 bis 4 C-Atomen substituiert sein kann, oder $R^2$ einen $-CO-R^3$-Rest bedeutet, wobei

$R^3$ einen gegebenenfalls durch Halogen, Alkoxy mit 1 bis 2 C-Atomen, Oxo (= O) oder Carboxyalkyl mit 2 bis 5 C-Atomen substituierten Alkylrest mit 1 bis 4 C-Atomen bedeutet, sowie deren Säureadditionssalze und Metallkomplexsalze.

Die Verbindungen der Formel I enthalten chirale Zentren und werden im allgemeinen in Form von Racematen oder als Diastereomerengemische erhalten. Die Diastereomeren lassen sich beispielsweise durch Säulenchromatographie trennen oder aufgrund von Löslichkeitsunterschieden in reiner Form isolieren. Aus solchen einheitlichen Diastereomeren kann man mit bekannten Methoden einheitliche Enantiomere erhalten. Diese werden von der vorliegenden Erfindung ebenfalls umfasst. Für die Anwendung der erfindungsgemässen Verbindungen als Fungizide sind sowohl die einheitlichen Diastereomeren bzw. Enantiomeren wie auch deren bei der Synthese anfallenden Gemische geeignet. Bevorzugt werden die letzteren verwendet.

X bedeutet beispielsweise 5-Fluor, 6-Fluor, 5-Chlor, 6-Chlor, 5-Brom, 6-Brom, 5-Methyl, 7-Methyl, 5-Methoxy, 6-Methoxy, 5-Trifluormethyl, 6-Trifluormethyl, 5-tert.-Butyl, 6-tert.-Butyl, 5-Nitro, 6-Nitro, 4,6-Dichlor, 5,7-Dichlor, 5,6-Dichlor, 5-Brom-7-chlor, 5-Chlor-7-brom, 5-Chlor-7-methyl, 5-Methyl-7-chlor, 4,5,7-Trichlor, 5,7-Dimethyl, 4,6-Dimethyl, 5-Ethoxy, 5-n-Butoxy und 5-Phenoxy.

R und $R^1$ bedeuten zum Beispiel Wasserstoff, Methyl, Ethyl, n-Propyl, Isopropyl und n-Butyl.

$R^2$ bedeutet beispielsweise Wasserstoff, Methyl, Ethyl, n-Propyl, n-Butyl, 4-Chlorbutyl, n-Pentyl, n-Hexyl, Allyl, 1-Buten-2-yl, Propargyl, Benzyl, 4-Fluorbenzyl, 4-Chlorbenzyl, 4-Brombenzyl, 2,4-Dichlorbenzyl und 4-Trifluormethylbenzyl.

$R^3$ bedeutet vorzugsweise Methyl, Ethyl, n-Propyl, Isopropyl, Chlormethyl, Chlorpropyl, Methoxymethyl, Acetonyl ($CH_3COCH_2$), Vinyl und Propenyl.

Geeignete Säureadditionssalze sind beispielsweise die Bromide, Chloride, Sulfate, Nitrate, Phosphate, Oxalate, oder Dodecylsulfonate. Die Wirksamkeit der Salze geht auf das Kation zurück, so dass die Wahl des Anions, soweit es pflanzenverträglich ist, beliebig ist. Metallkomplexsalze sind beispielsweise die Komplexe mit Kupfersulfat, Kobaltsulfat, Nickelchlorid oder Eisenchlorid.

Gegenstand der Erfindung ist auch ein Verfahren zur Herstellung der erfindungsgemässen Verbindungen der Formel I, welches darin besteht,

dass man ein 2,3-Dihydrobenzofuran-2-methylhalogenid der Formel II

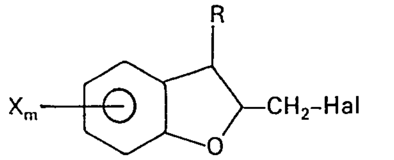

(II)

worin R, X und m die oben angegebenen Bedeutungen haben und Hal Chlor, Brom oder Jod bedeutet, mit einem Keton der Formel III

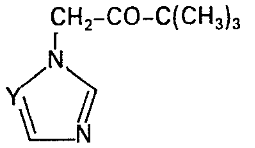

(III)

worin Y die oben genannte Bedeutung hat oder mit dessen Alkalienolat umsetzt und die so erhaltenen Verbindungen gegebenenfalls anschliessend mit Grignard-Reagentien versetzt oder reduziert und - falls gewünscht - danach verethert oder verestert.

Das Verfahren zur Herstellung der Ketone der Formel I, in denen Z eine CO-Gruppe darstellt, besteht darin, dass man bekannte Ketone der Formel III (DE-OS 26 38 479) oder deren Alkalienolate mit 2,3-Dihydrobenzofuran-2-methylhalogeniden der Formel II (s. Toyoshima u. Mitarb., Yakugaku Zasshi. 88, 503 [1968]), gegebenenfalls in Gegenwart einer starken anorganischen oder organischen Base, gegebenenfalls in Gegenwart eines Lösungs- oder Verdünnungsmittels, die Ein- oder Zweiphasensysteme bilden und gegebenenfalls unter Zusatz eines Reaktionsbeschleunigers und/oder Phasentransferkatalysators alkyliert, beispielsweise entsprechend der folgenden Umsetzung.

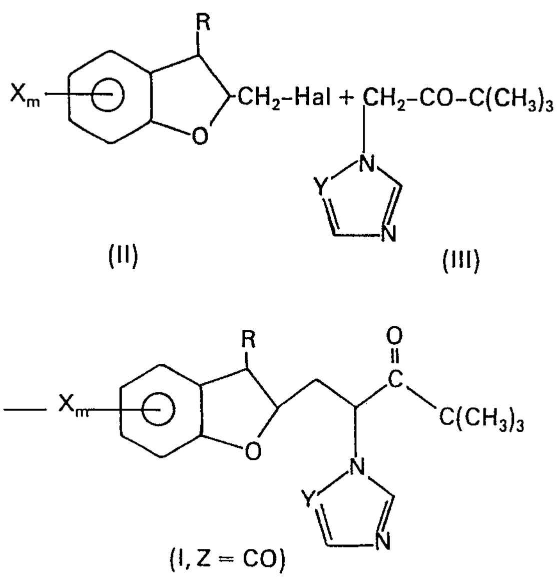

(II) (III)

(I, Z = CO)

Hierzu können die Ketone III zunächst zu den Alkalienolaten metalliert werden, indem man sie vorzugsweise in Gegenwart eines polaren aprotischen Lösungsmittels wie Dimethylformamid, Dimethylacetamid, Acetonitril, Sulfolan, Tetrahydrofuran oder Dimethoxyethan mit 1 bis 4 Äquivalenten, bevorzugt 1,0 Äquivalenten Metallierungsreagens wie Natriumhydrid, Lithiumdiisopropylamid, Calciumhydrid oder Kalium-tert.-Butoxid bei −10 bis 100°C, vorzugsweise bei 0 bis 70°C umsetzt. Nach anschliessender Zugabe von 0,8 bis 2,0, bevorzugt 1,0 Äquivalenten des jeweiligen Benzofuran-1-methylhalogenids II und gegebenenfalls eines Reaktionsbeschleunigers wie Natriumbromid, Natriumjodid, Kaliumbromid, Kaliumjodid oder eines Kronenethers wie 14-Krone-5, 18-Krone-6 oder Dibenzo-18-Krone-6 erhält man bei Reaktionstemperaturen zwischen 0 und 100°C, bevorzugt bei 3 bis 60°C die Ketone der Formel I.

Die erfindungsgemässen Ketone der Formel I (Z = CO) lassen sich gegebenenfalls in Gegenwart eines Lösungs- oder Verdünnungsmittels, bei Temperaturen zwischen −20 und 150°C, bei Normaldruck oder unter erhöhtem Druck mit Wasserstoff in Gegenwart eines Hydrierungskatalysators, mit Alkalihydrid oder komplexen Bor- bzw. Aluminiumhydriden, mit Aluminiumisopropoxid und Isopropanol, mit Natriumdithionit oder elektrochemisch zu den sekundären Alkoholen de Formel I, in der Z die Gruppe -CH- bedeutet, reduzieren.
 |
 OH

Für den Fall, dass X einen Alkenylrest bedeutet, wird die Reduktion mit den oben genannten Reduktionsmitteln ausser Wasserstoff in Gegenwart eines Hydrierungskatalysators durchgeführt.

Geeignete Lösungs- oder Verdünnungsmittel hierfür sind beispielsweise Wasser, Methanol, Ethanol, Isopropanol, Essigsäure, Diethylether, Tetrahydrofuran, Dioxan, Essigester, Toluol, Dimethylformamid oder entsprechende Gemische.

Zur katalytischen Hydrierung verwendet man Platin- oder Palladiumkatalysatoren auf inerten Trägern und hydriert bei Drucken von 20 bis 100 bar bis keine Wasserstoffaufnahme mehr erfolgt.

Für die als Reduktionsmittel verwendbaren Hydride seien beispielsweise Natriumhydrid oder Lithiumaluminiumhydrid genannt.

Die erfindungsgemässen Ketone der Formel I (Z = CO) lassen sich schliesslich mit einer Grignardverbindung der Formel

$R^1MgHal$ (IV)

in der $R^1$ $C_1$–$C_4$-Alkyl bedeutet und in der Hal Chlor, Brom oder Jod bedeutet, gegebenenfalls in Gegenwart eines Lösungs- oder Verdünnungsmittels und gegebenenfalls in Gegenwart eines Magnesium- oder Tetraalkylammonium-halogenids bei Temperaturen zwischen 0 und 100°C zu den tertiären Alkoholen der Formel I in der Z die Gruppe

OH
 |
-C-
 |
$R^1$

darstellt, alkylieren.

Als Lösungsmittel kommen bevorzugt Ether in Frage, wie Diethylether, Di-n-propylether, Methyl-tert.-butylether, Tetrahydrofuran, Dimethoxyethan oder Anisol, ferner tertiäre Amine wie N,N-Dimethylanilin, 1,2-Bis-(dimethylamino)-ethan sowie Phosphorsäure-tris(dimethylamid); gegebenenfalls kann man die Reaktion auch im Gemisch dieser Lösungsmittel mit aliphatischen oder aromatischen Kohlenwasserstoffen wie n-Pentan, n-Hexan, Cyclohexan, Benzol oder Toluol durchführen. Als ausbeutesteigernde Salze, die die üblichen Nebenreaktionen unterdrücken, kommen insbesondere wasserfreie Magnesiumhalogenide wie wasserfreies Magnesiumbromid oder wasserfreie Tetraalkylammoniumhalogenide wie z.B. Tetra-n-butyl-ammoniumchlorid in Frage. Die Reaktionstemperaturen lassen sich je nach Lösungsmittel zwischen 0 und 100°C variieren, bevorzugt sind Temperaturen zwischen 0 und 60°C. Die hierbei entstandenen Magnesiumalkoholate werden anschliessend durch Hydrolyse mit verdünnten wässrigen Säuren, wie Salzsäure, Schwefelsäure oder bevorzugt Essigsäure oder besonders bevorzugt mit wässriger Ammoniumchloridlösung in die Alkohole übergeführt und diese nach Entfernen der wässrigen Phase, falls gewünscht, in üblicher Weise durch Extraktion, Umkristallisieren oder Chromatographie gereinigt.

Die so erhaltenen Alkohole der Formel I

(Z = -CH-OH- und

OH
|
-C-)
|
$R^1$

lassen sich mit Alkylierungsmitteln der Formel V

$R^2$ - Hal (V)

worin $R^2$ und Hal die oben angegebenen Bedeutungen haben, gegebenenfalls in Gegenwart eines Lösungs- oder Verdünnungsmittels die Ein- oder Zweiphasensysteme bilden, gegebenenfalls unter Zusatz einer anorganischen Base und gegebenenfalls unter Zusatz eines Reaktionsbeschleunigers und/oder eines Phasentransferkatalysators zu den entsprechenden Verbindungen, in denen Z -CH-$OR^2$- oder -$R^1$C-$OR^2$- bedeutet, verethern.

Hierfür eignen sich beispielsweise folgende Lösungs- oder Verdünnungsmittel: Diethylether, Tetrahydrofuran, Dioxan, n-Pentan, monohalogenierte Kohlenwasserstoffe mit 2 bis 6 C-Atomen wie beispielsweise Chlorethan, Bromethan, 1-Chlorpropan, 1-Brompropan, 1-Chlorbutan, 1-Brombutan, 1-Chlorpentan, 1-Brompentan, 1-Chlorhexan, 1-Bromhexan, ferner Cyclohexan, Methylenchlorid, Chloroform, Toluol, Chlorbenzol, Xylol oder Dimethylformamid.

Als anorganische Basen seien beispielsweise genannt: Alkali- und Erdalkalihydroxide wie Natriumhydroxid, Kaliumhydroxid, Calciumhydroxid, Bariumhydroxid, Alkali- und Erdalkalicarbonate wie Natriumcarbonat, Kaliumcarbonat, Natriumhydrogencarbonat, Kaliumhydrogencarbonat, Calciumcarbonat, Bariumcarbonat, Alkali- oder Erdalkalialkoholate wie Natriummethylat, Natriumethylat,Magnesiummethylat,Natriumisopropylat oder Kalium-tert.-butylat.

Geeignete Reaktionsbeschleuniger sind beispielsweise Metallhalogenide wie Natriumbromid, Natriumjodid, Kaliumbromid, Kaliumjodid und Kronenether wie 14-Krone-5, 18-Krone-6, Dibenzo-18-Krone-6 oder Dicyclohexano-18-Krone-6.

Als Phasentransferkatalysatoren kommen vorzugsweise quaternäre Ammoniumsalze wie Tetrabutylammoniumchlorid, -bromid, -iodid oder -hydrogensulfat, Benzyl-triethylammoniumchlorid, Methyl-trioctylammoniumchlorid und -bromid und Phosphoniumsalze wie Tetrabutylphosphoniumbromid und -iodid in Frage.

Die Veresterung der Alkohole der Formel I

(Z = CHOH und

OH
|
-C-)
|
$R^1$

kann mit Säurechloriden oder -anhydriden der Formeln

$R^3$-CO-Cl (VI) oder $(R^3\text{-CO})_2O$ (VII)

gegebenenfalls in Gegenwart eines Lösungs- oder Verdünnungsmittels, gegebenenfalls unter Zusatz eines säurebindenden Mittels und gegebenenfalls unter Zusatz eines Reaktionsbeschleunigers erfolgen. Hierfür eignen sich dieselben Basen wie für die Veretherung. Zusätzlich können tertiäre Amine wie Trimethylamin, Triethylamin, N,N-Dimethylanilin, N,N-Dimethylcyclohexylamin, N-Methylpiperidin oder Pyridin als Basen und Imidazol, N-Methylimidazol, 1,2,4-Triazol, 4-Dimethylaminopyridin und 4-Pyrrolidinopyridin als Reaktionsbeschleuniger verwendet werden.

Mit geeigneten Basen z.B. Alkalihydrid wie Natriumhydrid oder Alkali- oder Erdalkalialkoholaten wie Natriummethylat können die Alkohole auch in einer ersten Umsetzung zunächst in ihre Alkoholat-Salze überführt werden und dann als solche zur Reaktion gebracht werden.

Die so erhaltenen Verbindungen der Formel I, in denen Z -CHO-CO-$R^3$- oder -$R^1$CO-CO-$R^3$ bedeutet, werden nach üblichen Methoden isoliert, gegebenenfalls gereinigt und gegebenenfalls mit Säuren oder Metallsalzen zu Salzen bzw. zu Metallkomplexen umgesetzt.

Als Beispiele für die neuen erfindungsgemässen Verbindungen der Formel I seien im einzelnen genannt:

1-(2′,3′-Dihydrobenzofuran-2′-yl)-2-(1,2,4-triazol-1-yl)-4,4-dimethylpentan-3-on

1-(2′,3′-Dihydrobenzofuran-2′-yl)-2-(1,2,4-triazol-1-yl)-4,4-dimethylpentan-3-ol
1-(2′,3′-Dihydrobenzofuran-2′-yl)-2-(1,2,4-triazol-1-yl)-3-methoxy-4,4-dimethylpentan
1-(2′,3′-Dihydrobenzofuran-2′-yl)-2-(1,2,3-triazol-1-yl)-3-(1-propyloxy)-4,4-dimethylpentan
1-(2′,3′-Dihydrobenzofuran-2′-yl)-2-(1,2,4-triazol-1-yl)-3,4,4-trimethylpentan-3-ol
1-(2′,3′-Dihydrobenzofuran-2′-yl)-2-(1,2,4-triazol-1-yl)-3-methoxy-3,4,4-trimethylpentan
1-(2′,3′-Dihydrobenzofuran-2′-yl)-2-(1,2,4-triazol-1-yl)-3-allyloxy-4,4-dimethylpentan
1-(2′,3′-Dihydrobenzofuran-2′-yl)-2-(1,2,4-triazol-1-yl)-3-(4-chlor-1-butoxy)-4,4-dimethylpentan
1-(2′,3′-Dihydrobenzofuran-2′-yl)-2-(1,2,4-triazol-1-yl)-3-(1-pentyloxy)-4,4-dimethylpentan
1-(2′,3′-Dihydrobenzofuran-2′-yl)-2-(1,2,4-triazol-1-yl)-3-benzyloxy-4,4-dimethylpentan
1-(2′,3′-Dihydrobenzofuran-2′-yl)-2-(1,2,4-triazol-1-yl)-3-(4-chlorbenzyloxy)-4,4-dimethylpentan
1-(2′,3′-Dihydrobenzofuran-2′-yl)-2-(1,2,4-triazol-1-yl)-3-(4-Nitrobenzyloxy)-4,4-dimethylpentan
1-(2′,3′-Dihydrobenzofuran-2′-yl)-2-(1,2,4-triazol-1-yl)-3-(4-trifluormethylbenzyloxy)-4,4-dimethylpentan
1-(2′,3′-Dihydrobenzofuran-2′-yl)-2-(1,2,4-triazol-1-yl)-3-(propyloxy)-4,4-dimethylpentan
1-(2′,3′-Dihydrobenzofuran-2′-yl)-2-(1,2,4-triazol-1-yl)-3-acetoxy-4,4-dimethylpentan
1-(2′,3′-Dihydrobenzofuran-2′-yl)-2-(1,2,4-triazol-1-yl)-3-acetoxy-3,4,4-trimethylpentan
1-(2′,3′-Dihydrobenzofuran-2′-yl)-2-(1,2,4-triazol-1-yl)-3-propionyloxy-4,4-dimethylpentan
1-(2′,3′-Dihydrobenzofuran-2′-yl)-2-(1,2,4-triazol-1-yl)-3-chloracetoxy-4,4-dimethylpentan
1-(2′,3′-Dihydrobenzofuran-2′-yl)-2-(1,2,4-triazol-1-yl)-3-bromacetoxy-4,4-dimethylpentan
1-(2′,3′-Dihydrobenzofuran-2′-yl)-2-(1,2,4-triazol-1-yl)-3-methoxyacetyl-4,4-dimethylpentan
1-(2′,3′-Dihydrobenzofuran-2′-yl)-2-(1,2,4-triazol-1-yl)-3-acetoacetoxy-4,4-dimethylpentan
1-(2′,3′-Dihydro-3′-methylbenzofuran-2′-yl)-2-(1,2,4-triazol-1-yl)-4,4-dimethylpentan-3-on
1-(2′,3′-Dihydro-3′-methylbenzofuran-2′-yl)-2-(1,2,4-triazol-1-yl)-4,4-dimethylpentan-3-ol
1-(2′,3′-Dihydro-3′-methylbenzofuran-2′-yl)-2-(1,2,4-triazol-1-yl)-3-methoxy-4,4-dimethylpentan
1-(2′,3′-Dihydro-3′-methylbenzofuran-2′-yl)-2-(1,2,4-triazol-1-yl)-3-acetoxy-4,4-dimethylpentan
1-(2′,3′-Dihydro-3′-methylbenzofuran-2′-yl)-2-(1,2,4-triazol-1-yl)-3-propionyloxy-4,4-dimethylpentan
1-(2′,3′-Dihydro-5′-fluorbenzofuran-2′-yl)-2-(1,2,4-triazol-1-yl)-4,4-dimethylpentan-3-on
1-(2′,3′-Dihydro-5′-methylbenzofuran-2′-yl)-(1,2,4-triazol-1-yl)-4,4-dimethylpentan-3-on
1-(2′,3′-Dihydro-5′-methylbenzofuran-2′-yl)-(1,2,4-triazol-1-yl)-4,4-dimethylpentan-3-ol
1-(2′,3′-Dihydro-5′-methylbenzofuran-2′-yl)-(1,2,4-triazol-1-yl)-3-acetoxy-4,4-dimethylpentan
1-(2′,3′-Dihydro-5′-methylbenzofuran-2′-yl)-2-(1,2,4-triazol-1-yl)-3-propionyloxy-4,4-dimethylpentan
1-(2′,3′-Dihydro-5′-tert.-butylbenzofuran-2′-yl)-2-(1,2,4-triazol-1-yl)-4,4-dimethylpentan-3-on
1-(2′,3′-Dihydro-5′-tert.-butylbenzofuran-2′-yl)-2-(1,2,4-triazol-1-yl)-4,4-dimethylpentan-3-ol
1-(2′,3′-Dihydro-5′-tert.-butylbenzofuran-2′-yl)-2-(1,2,4-triazol-1-yl)-3,4,4-trimethylpentan-3-ol
1-(2′,3′-Dihydro-5′,7′-dimethylbenzofuran-2′-yl)-2-(1,2,4-triazol-1-yl)-4,4-dimethylpentan-3-on
1-)2′,3′-Dihydro-5′,7′-dimethylbenzofuran-2′-yl)-2-(1,2,4-triazol-1-yl)-4,4-dimethylpentan-3-ol
1-(2′,3′-Dihydro-5′,7′-dichlorbenzofuran-2′-yl)-2-(1,2,4-triazol-1-yl)-4,4-dimethylpentan-3-on
1-(2′,3′-Dihydro-5′,7′-dichlorbenzofuran-2′-yl)-2-(1,2,4-triazol-1-yl)4,4-dimethylpentan-3-ol
1-(2′,3′-Dihydro-5′,7′-dichlorbenzofuran-2′-yl)-2-(1,2,4-triazol-1-yl)-3,4,4-trimethylpentan-3-ol
1-(2′,3′-Dihydro-5′,7′-dichlorbenzofuran-2′-yl)-2-(1,2,4-triazol-1-yl)-3-methoxy-4,4-dimethylpentan
1-(2′,3′-Dihydro-5′,7′-dichlorbenzofuran-2′-yl)-2-(1,2,4-triazol-1-yl)-3-(1-propyloxy)-4,4-dimethylpentan
1-(2′,3′-Dihydro-5′,7′-dichlorbenzofuran-2′-yl)-2-(1,2,4-triazol-1-yl)-3-allyloxy-4,4-dimethylpentan
1-(2′,3′-Dihydro-5′,7′-dichlorbenzofuran-2′-yl)-2-(1,2,4-triazol-1-yl)-3-(2,4-dichlorbenzyloxy)-4,4-dimethylpentan
1-(2′,3′-Dihydro-5′,7′-dichlorbenzofuran-2′-yl)-2-(1,2,4-triazol-1-yl)-3-(4-fluorbenzyloxy)-4,4-dimethylpentan
1-(2′,3′-Dihydro-5′,7′-dichlorbenzofuran-2′-yl)-2-(1,2,4-triazol-1-yl)-3-(4-methylbenzyloxy)-4,4-dimethylpentan
1-(2′,3′-Dihydro-5′,7′-dichlorbenzofuran-2′-yl)-2-(1,2,4-triazol-1-yl)-3-acetoxy-4,4-dimethylpentan
1-(2′,3′-Dihydro-3′-methyl-5′,7′-dichlorbenzofuran-2′-yl)-2-(1,2,4-triazol-1-yl)-4,4-dimethylpentan-3-on
1-(2′,3′-Dihydro-3′-methyl-5′,7′-dichlorbenzofuran-2′-yl)-2-(1,2,4-triazol-1-yl)-4,4-dimethylpentan-3-ol
1-(2′,3′-Dihydro-3′-methyl-5′,7′-dichlorbenzofuran-2′-yl)-2-(1,2,4-triazol-1-yl)-3,4,4-trimethylpentan-3-ol
1-(2′,3′-Dihydro-3′-methyl-5′,7′-dichlorbenzofuran-2′-yl)-2-(1,2,4-triazol-1-yl)-3-methoxy-4,4-dimethylpentan
1-(2′,3′-Dihydro-4′,6′-dichlorbenzofuran-2′-yl)-2-(1,2,4-triazol-1-yl)-4,4-dimethylpentan-3-on
1-(2′,3′-Dihydro-4′,6′-dichlorbenzofuran-2′-yl)-2-(1,2,4-triazol-1-yl)-4,4-dimethylpentan-3-ol
1-(2′,3′-Dihydro-4′,6′-dichlorbenzofuran-2′-yl)-2-(1,2,4-triazol-1-yl)-3-methoxy-4,4-dimethylpentan
1-(2′,3′-Dihydro-4′,6′-dichlorbenzofuran-2′-yl)-2-(1,2,4-triazol-1-yl)-3-(1-butoxy)-4,4-dimethylpentan
1-(2′,3′-Dihydro-4′,6′-dichlorbenzofuran-2′-yl)-2-(1,2,4-triazol-1-yl)-3-acetoxy-4,4-dimethylpentan
1-(2′,3′-Dihydro-5′-brom-7′-chlorbenzofuran-2′-yl)-2-(1,2,4-triazol-1-yl)-4,4-dimethylpentan-3-on
1-(2′,3′-Dihydro-5′-brom-7′-chlorbenzofuran-2'-yl)-2-(1,2,4-triazol-1-yl)-4,4-dimethylpentan-3-ol
1-(2',3'-Dihydro-5'-brom-7'-chlorbenzofuran-2'-yl)-2-(1,2,4-triazol-1-yl)-3-methoxy-4,4-dimethylpentan
1-(2',3'-Dihydro-5'-chlor-7'-methylbenzofuran-2'-yl)-2-(1,2,4-triazol-1-yl)-4,4-dimethylpentan-3-on
1-(2',3'-Dihydro-5'-chlor-7'-methylbenzofuran-2'-yl)-2-(1,2,4-triazol-1-yl)-4,4-dimethylpentan-3-ol
1-(2',3'-Dihydro-5'-chlor-7'-methylbenzo-furan-2'-yl)-2-(1,2,4-triazol-1-yl)-3-propionyloxy-4,4-dimethylpentan
1-(2',3'-Dihydro-5'-nitrobenzofuran-2'-yl)-2-(1,2,4-triazol-1-yl)-4,4-dimethylpentan-3-on
1-(2',3'-Dihydro-5'-nitrobenzofuran-2'-yl)-2-(1,2,4-triazol-1-yl)-4,4-dimethylpentan-3-ol
1-(2',3'-Dihydro-5'-ethoxybenzofuran-2'-yl)-2-(1,2,4-triazol-1-yl)-4,4-dimethylpentan-3-on
1-(2',3'-Dihydro-5'-ethoxybenzofuran-2'-yl)-2-(1,2,4-triazol-1-yl)-4,4-dimethylpentan-3-ol
1-(2',3'-Dihydro-5'-trifluormethylbenzofuran-2'-yl)-2-(1,2,4-triazol-1-yl)-4,4-dimethylpentan-3-on
1-(2',3'-Dihydro-5'-trifluormethylbenzofuran-2'-yl)-2-(1,2,4-triazol-1-yl)-4,4-dimethylpentan-3-ol
1-(2',3'-Dihydro-4',5',7'-trichlorbenzofuran-2'-yl)-2-(1,2,4-triazol-1-yl)-4,4-dimethylpentan-3-ol
1-(2',3'-Dihydro-4',5',7'-trichlorbenzofuran-2'-yl)-2-(1,2,4-triazol-1-yl)-4,4-dimethylpentan-3-on

Die Herstellung der erfindungsgemässen Verbindungen der Formel I wird durch folgende Beispiele erläutert.

Beispiel 1

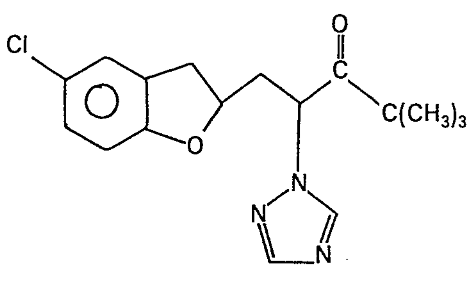

Zu einer gerührten Suspension von 9,6 g (0,4 Mol) Natriumhydrid in 120 ml trockenem Dimethylformamid (DMF) wird die Lösung von 66,7 g (0,4 Mol) 2,2-Dimethyl-4-(1,2,4-triazol-1-yl)-butan-3-on (siehe DE-OS 26 38 470) in 80 ml DMF unter einer trockenen Stickstoffatmosphäre bei 20 bis 25°C zugetropf. Nach dreistündigem Rühren wird bei 25°C die Lösung von 99 g (0,4 Mol) 2,3-Dihydro-2-brommethyl-5-chlorbenzofuran (siehe S. Toshima u. Mitarb., Yakugaku Zasshi., 88, 503 [1968]) in 80 ml DMF zugetropft und das Reaktionsgemisch weitere 48 Stunden nachgerührt. 50 ml Eiswasser werden vorsichtig zugetropft und das Gemisch wird im Vakuum eingeengt. Der Rückstand wird zwischen 50 ml Methylenchlorid und 200 ml Wasser verteilt, die organische Phase zweimal mit je 200 ml Wasser gewaschen, über $Na_2SO_4$ getrocknet und eingeengt. Der Rückstand wird mit 50 ml Petrolether bei 10°C 1 Stunde gerührt und der Niederschlag abgesaugt. Man erhält 55,4 g (41,5% d.Th.) 1-(2',3'-Dihydro-5'-chlorbenzofuran -2'-yl) -2-(1,2,4-triazol-1-yl)-4,4 -dimenthylpentan-3-on als weisse Kristalle vom Schmelzpunkt 131 bis 134°C (Wirkstoff Nr. 1).

Beispiel 2

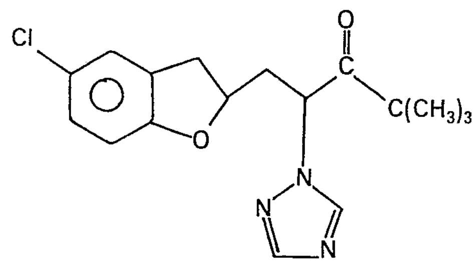

Zu einer Lösung von 35,3 g (0,105 Mol) 1-(2',3'-Dihydro-5'-chlordibenzofuran-2'-yl)-2-(1,2,4-triazol-1-yl)-4,4-dimethylpentan-3-on in 250 ml Methanol werden zwischen 0 und +5°C 4,6 g (0,12 Mol) Natriumborhydrid portionsweise zugegeben. Nach 12stündigem Rühren bei 20°C wird das Gemisch eingeengt. Der Rückstand wird mit 150 ml 20%iger (Gew.-%) Kalilauge und mit 400 ml Methylenchlorid 1 Std. gerührt. Die organische Phase wird zweimal mit je 70 ml Wasser gewaschen, über Natriumsulfat getrocknet und eingeengt. Der Rückstand kristallisiert bei 10°C nach Zugabe von 70 ml Petrolether. Man isoliert 33,9 g (96,2% d.Th.) 1-(2',3'-Dihydro-5'-chlorbenzofuran-2'-yl)-2-(1,2,4-triazol-1-yl)-4,4-dimethylpentan-3-ol als farblose Kristalle vom Schmelzpunkt 63 bis 66°C (Wirkstoff Nr. 2).

Beispiel 3

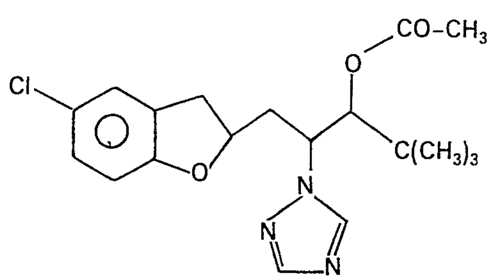

Eine Mischung aus 12,5 g (0,0375 Mol) 1-(2',3'-Dihydro-5'-chlorbenzofuran-2'-yl)-2-(1,2,4-triazol-1-yl)-4,4-dimethyl-pentan-3-ol, 4,6 g (0,0375 Mol) 4-Dimethylaminopyridin und 100 ml Acetanhydrid werden 12 Stunden bei 60°C gerührt und

anschliessend im Vakuum eingeengt. Der Rückstand wird mit 300 ml Ether und 100 ml einer 6%igen Natriumhydrogencarbonatlösung 1½ Stunden gerührt, die organische Phase abgetrennt, über Natriumsulfat getrocknet und eingeengt. Der feste, farblose Rückstand wird mit n-Pentan gerührt und abgesaugt.

Man erhält 10,5 g (74,2% d.Th.) 1-(2′,3′-Dihydro-5′-chlorbenzofuran-2′-yl)-2-(1,2,4-triazol-1-yl)-3-acetoxy-4,4-dimethylpentan vom Schmelzpunkt 98 bis 99°C (Wirkstoff Nr.3).

Beispiel 4

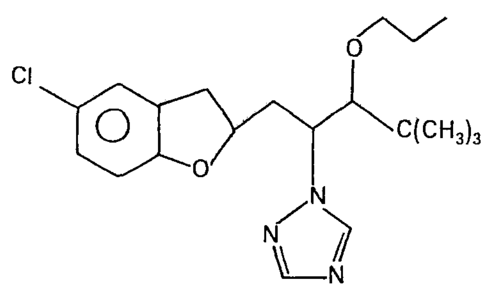

Unter kräftigem Rühren wird eine Mischung aus 12,5 g (0,0375 Mol) 1-(2′,3′-Dihydro-5′-chlorbenzofuran-2′-yl)-2-(1,2,4-triazolyl)-4,4-dimethylpentan-3-ol, 100 ml 1-Chlorpropan, 4 g Tetrabutylammoniumhydrogensulfat und 65 g 50%iger wässriger Natriumhydroxidlösung 24 Stunden auf 40°C erwärmt. Die Mischung wird sodann mit 300 ml Wasser versetzt und fünfmal mit je 80 ml Methylenchlorid extrahiert. Die vereinigten Extrakte werden fünfmal mit je 100 ml Wasser ausgeschüttelt, über Magnesiumsulfat getrocknet und im Vakuum - schliesslich bis 80°C und 0,1 mbar - eingeengt. Der ölige Rückstand wird mit 30 ml Petrolether 3 Tage bei +5°C im Kühlschrank stehen gelassen. Die ausgefallenen, farblosen Kristalle werden abgesaugt, mit Pentan gewaschen und getrocknet.

Man erhält 9,8 g (69,2% d.Th.) 1-(2′,3′-Dihydro-5′-chlorbenzofuran-2′-yl)-2-(1,2,4-triazol-1-yl)-3-(1-propyloxy)-4,4-dimethylpentan vom Schmelzpunkt 75 bis 78°C (Wirkstoff Nr. 4).

Beispiel 5

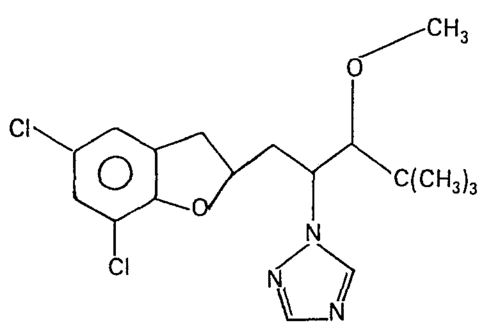

Zu einer Suspension von 2,4 g (0,1 Mol) Natriumhydrid in 100 ml trockenem Tetrahydrofuran wird eine Lösung von 22 g (0,06 Mol) 1-(2′,3′-Dihydro-5′,7′-dichlorbenzofuran-2′-yl)-2-(1,2,4-triazol-1-yl)-4,4,-dimethylpentan-3-ol (Beispiel 17) in 60 ml Tetrahydrofuran getropft. Nach 12stündigem Rühren bei Raumtemperatur wird das Gemisch mit 14,2 g (0,1 Mol) Iodmethan versetzt. Nach 20stündigem Rühren wird das Reaktionsgemisch vorsichtig mit 40 ml Wasser zersetzt und eingeengt. Der Rückstand wird in 350 ml Ether aufgenommen, zweimal mit je 100 ml Wasser gewaschen, die Etherphase getrocknet und eingeengt. Der Rückstand wird mit 50 ml Petrolether bei 5°C 1 Stunde gerührt. Der kristalline, farblose Niederschlag wird abgesaugt, mit Petrolether gewaschen und getrocknet.

Man erhält 11,4 g (49,5% d.Th.) 1-(2′,3′-Dihydro-5′,7′-dichlorbenzofuran-2′-yl)-2-(1,2,4-triazol-1-yl)-3-methoxy-4,4-dimethylpentan vom Schmelzpunkt 117 bis 118°C (Wirkstoff Nr. 5).

Beispiel 6

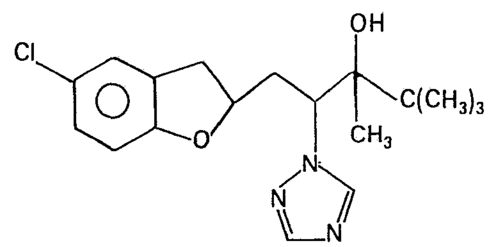

Zu einer Lösung von 5,4 g (0,0406 Mol) Methylmagnesiumbromid in 30 ml Tetrahydrofuran tropft man eine Lösung von 7 g (0,021 Mol) 1-(2′,3′-Dihydro-5′-chlorbenzofuran-2′-yl)-4,4-dimethyl-pentan-3-on (Beispiel 1) in 50 ml Tetrahydrofuran und rührt 20 Stunden bei Raumtemperatur. Die Mischung wird sodann in 800 ml 10%iger wässriger Ammoniumchloridlösung eingerührt und zweimal mit je 200 ml Dichlormethan extrahiert. Nach dem Waschen der organischen Phase und Trocknen über Magnesiumsulfat wird das Dichlormethan im Vakuum abgedampft. Der Rückstand kristallisiert bei +5°C nach Zugabe von 10 ml Petrolether und 10 ml Ether. Man isoliert 3,2 g (43,6% d.Th.) 1-(2′,3′-Dihydro-5′-chlorbenzofuran-2′-yl)-2-(1,2,4-triazol-1-yl)-3,4,4-trimethylpentan-3-ol als farblose Kristalle vom Schmelzpunkt 144 bis 146°C (Wirkstoff Nr. 6).

Weitere Beispiele für die erfindungsgemässen Verbindungen der Formel (I) sind in der folgenden Tabelle 1 enthalten.

| Beispiel Nummer | $X_m$ | R | Y | Z | Fp. (°C) | IR (Film) [$cm^{-1}$] |
|---|---|---|---|---|---|---|
| 7 | 5-Cl | H | CH | CO | Harz | 3104, 2970, 1703, 1465, 1225, 1215, 1162, 1100, 802, 732. |
| 8 | 5-Cl | H | CH | CHOH | 163–165 | |
| 9 | 5-Cl | $CH_3$ | N | CO | 128–130 | |
| 10 | 5-Cl | $CH_3$ | N | CHOH | Harz | 2955, 1492, 1464, 1265, 1220, 1128, 1008, 965, 805, 672. |
| 11 | 5-$CH_3$-O | H | N | CO | 120–122 | |
| 12 | 5-$CH_3$ | H | N | CO | Harz | 3110, 2970, 1708, 1480, 1360, 1268, 1230, 1200, 1128, 802. |
| 13 | 5-$CH_3$ | H | N | CHOH | Harz | 3300, 2970, 1610, 1485, 1270, 1200, 1010, 982, 805, 680. |
| 14 | 5-tert.-$C_4H_9$ | H | N | CO | Harz | 3120, 2968, 1710, 1490, 1360, 1270, 1230, 1135, 818, 680. |
| 15 | 5,7-$Cl_2$ | H | N | CO | Harz | 3105, 2956, 1700, 1566, 1445, 1262, 1128, 992, 850, 756. |
| 16 | 5,7-$Cl_2$ | H | N | CO | 155–159 | (Hydrochlorid). |
| 17 | 5,7-$Cl_2$ | H | N | CHOH (Diastereomer I) | 137–139 | |
| 18 | 5,7-$Cl_2$ | H | N | CHOH (Diastereomerengemisch) | Harz | 2950, 1458, 1270, 1192, 1130, 1008, 980, 850, 755, 672. |
| 19 | 5,7-$Cl_2$ | H | N | -CH-OCO-$CH_3$ | 99–101 | |
| 20 | 5,7-$Cl_2$ | H | N | -CH-O 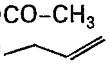 | 95–97 | |
| 21 | 5,7-$Cl_2$ | H | N | -CH-O 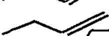 | 111–113 | |
| 22 | 5,7-$Cl_2$ | H | N | -CH-O-$CH_2$- 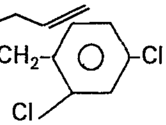 (Diastereomer I) | 146–148 | |
| 23 | 5,7-$Cl_2$ | H | N | -CH-O-$CH_2$- 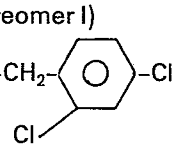 (Diastereomerengemisch) | Harz | 3122, 2950, 1576, 1456, 1212, 1122, 1068, 842, 752, 672. |
| 24 | 5,7-$Cl_2$ | $CH_3$ | N | CO | Harz | 3118, 2960, 1708, 1445, 1262, 1160, 1126, 962, 878, 848, 750. |
| 25 | 5,7-$Cl_2$ | $CH_3$ | N | CH-OH | Harz | 2960, 1495, 1450, 1268, 1190, 1162, 1128, 1010, 960, 850, 750. |
| 26 | 4,6-$Cl_2$ | H | N | CO | 90–92 | |
| 27 | 4,6-$Cl_2$ | H | N | CH-OH | 122–124 | |
| 28 | 4,6-$Cl_2$ | H | N | -CH-OCO$CH_3$ | 116–118 | |
| 29 | 4,6-$Cl_2$ | H | N | -CH-O 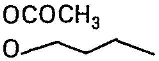 | 92–94. | |
| 30 | 5-Br, 7-Cl | H | N | CO | Harz | 3120, 2970, 1711, 1495, 1454, 1268, 1200, 1132, 854, 676. |
| 31 | 5-Br, 7-Cl | H | N | CHOH | Harz | 3350, 2960, 1496, 1454, 1270, 1130, 1082, 980, 852, 738. |
| 32 | 5-Cl, 7-$CH_3$ | H | N | CO | Harz | 3120, 2965, 1709, 1460, 1268, 1190, 1130, 948, 855, 676. |
| 33 | 5-Cl, 7-$CH_3$ | H | N | CHOH (Diastereomer I) | 145–147 | |
| 34 | 5-CL, 7-$CH_3$ | H | N | CHOH (Diastereomerengemisch) | Harz | 3200, 2980, 1510, 1470, 1280, 1200, 1135, 1088, 864, 788. |
| 35 | H | H | N | CO | Harz | 3120, 2965, 1708, 1588, 1470, 1360, 1268, 1220, 1130, 748. |
| 36 | H | H | N | CHOH | Harz | 3290, 2955, 1592, 1474, 1270, 1225, 1130, 866, 746, 675. |
| 37 | H | H | N | -CH-O 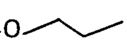 | Harz | 2958, 1592, 1472, 1268, 1225, 1128, 1090, 865, 746, 672. |
| 38 | H | H | N | -CH-O 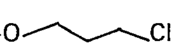 | Harz | 3115, 2950, 1592, 1473, 1225, 1130, 1096, 868, 748, 675. |

| Beispiel Nummer | $X_m$ | R | Y | Z | Fp. (°C) | IR (Film) [$cm^{-1}$] |
|---|---|---|---|---|---|---|
| 39 | H | H | N | -CH-O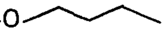 | Harz | 2958, 1590, 1490, 1472, 1276, 1225, 1130, 1090, 868, 749. |
| 40 | 5-tert.-$C_4H_9$ | H | N | CHOH | 126-128 | |
| 41 | 5-$CH_3O$ | H | N | CHOH | 95-97 | |

Die erfindungsgemässen Verbindungen und ihre Salze und Metallkomplexverbindungen zeichnen sich durch eine hervorragende Wirksamkeit gegen ein breites Spektrum von pflanzenpathogenen Pilzen, insbesondere aus der Klasse der Ascomyceten und Basidiomyceten, aus. Sie sind zum Teil systemisch wirksam und können als Blatt- und Bodenfungizide eingesetzt werden.

Besonders interessant sind die fungiziden Verbindungen für die Bekämpfung einer Vielzahl von Pilzen an verschiedenen Kulturpflanzen oder ihren Samen, insbesondere Weizen, Roggen, Gerste, Hafer, Reis, Mais, Baumwolle, Soja, Kaffee, Zuckerrohr, Obst und Zierpflanzen im Gartenbau, sowie in Gemüsen wie Gurken, Bohnen oder Kürbisgewächsen.

Die neuen Verbindungen sind insbesondere geeignet zur Bekämpfung folgender Pflanzenkrankheiten:

Erysiphe graminis (echter Mehltau) in Getreide, Erysiphe cichoracearum (echter Mehltau) an Kürbisgewächsen
Podosphaera leucotricha an Äpfeln,
Uncinula necator an Reben,
Erysiphe polygoni an Bohnen,
Sphaerotheca pannosa an Rosen,
Puccinia-Arten an Getreide,
Rhizoctonia solani an Baumwolle sowie Helminthosporiumarten an Getreide,
Ustilago-Arten an Getreide und Zuckerrohr,
Rhynchosporium secale an Getreide und ganz besonders Venturia inaequalis (Apfelschorf).

Die Verbindungen werden angewendet, indem man die Pflanzen mit den Wirkstoffen besprüht oder bestäubt oder die Samen der Pflanzen mit den Wirkstoffen behandelt. Die Anwendung erfolgt vor oder nach der Infektion der Pflanzen oder Samen durch die Pilze.

Die erfindungsgemässen Substanzen können in die üblichen Formulierungen überführt werden, wie Lösungen, Emulsionen, Suspensionen, Stäube, Pulver, Pasten und Granulate. Die Anwendungsformen richten sich ganz nach den Verwendungszwecken; sie sollen in jedem Fall eine feine und gleichmässige Verteilung der wirksamen Substanz gewährleisten. Die Formulierungen werden in bekannter Weise hergestellt, z.B. durch Verstrecken des Wirkstoffs mit Lösungsmitteln und/oder Trägerstoffen, gegebenenfalls unter Verwendung von Emulgiermitteln und Dispergiermitteln, wobei im Falle der Benutzung von Wasser als Verdünnungsmittel auch andere organische Lösungsmittel als Hilfslösungsmittel verwendet werden können. Als Hilfsstoffe kommen dafür im wesentlichen in Frage: Lösungsmittel wie Aromaten (z.B. Xylol, Benzol), chlorierte Aromaten (z.B. Chlorbenzole), Paraffine (z.B. Erdölfraktionen), Alkohole (z.B. Methanol, Butanol), Ketone (z.B. Cyclohexanon), Amine (z.B. Ethanolamin, Dimethylformamid) und Wasser; Trägerstoffe wie natürliche Gesteinsmehle (z.B. Kaoline, Tonerden, Talkum, Kreide) und synthetische Gesteinsmehle (z.B. hochdisperse Kieselsäure, Silikate); Emulgiermittel wie nichtionogene und anionische Emulgatoren (z.B. Polyoxyethylen-Fettalkohol-Ether, Alkylsulfonate und Arylsulfonate) und Dispergiermittel, wie Lignin, Sulfitablaugen und Methylcellulose.

Die fungiziden Mittel enthalten im allgemeinen zwischen 0,1 und 95, vorzugsweise zwischen 0,5 und 90 Gew.-% Wirkstoff.

Die Aufwandmengen liegen je nach Art des gewünschten Effektes zwischen 0,02 und 3 kg Wirkstoff oder mehr je ha. Die neuen Verbindungen können auch in Materialschutz u.a. zur Bekämpfung holzzerstörender Pilze wie Coniophora puteanea und Polystictus versicolor eingesetzt werden. Die neuen Wirkstoffe können auch als fungizid wirksame Bestandteile öliger Holzschutzmittel zum Schutz von Holz gegen holzverfärbende Pilze eingesetzt werden. Die Anwendung erfolgt in der Weise, dass man das Holz mit diesen Mitteln behandelt, beispielsweise tränkt oder anstreicht.

Die Mittel bzw. die daraus hergestellten gebrauchsfertigen Zubereitungen wie Lösungen, Emulsionen, Suspensionen, Pulver, Stäube, Pasten oder Granulate werden in bekannter Weise angewendet, beispielsweise durch Versprühen, Vernebeln, Verstäuben, Verstreuen, Beizen oder Giessen.

Beispiele für solche Zubereitungen sind:

I. Man vermischt 90 Gew.-Teile der Verbindung des Beispiels 1 mit 10 Gew.-Teilen N-Methyl-alpha-pyrrolidon und erhält eine Lösung, die zur Anwendung in Form kleinster Tropfen geeignet ist.

II. 10 Gew.-Teile der Verbindung des Beispiels 2 werden in einer Mischung gelöst, die aus 90 Gew.-Teilen Xylol, 6 Gew.-Teilen des Anlagerungsproduktes von 8 bis 10 Mol Ethylenoxid an 1 Mol Ölsäure-N-monoethanolamin, 2 Gew.-Teilen Calciumsalz der Dodecylbenzolsulfonsäure und 2 Gew.-Teilen des Anlagerungsproduktes von 40 Mol Ethylenoxid an 1 Mol Ricinusöl besteht. Durch Ausgiessen und feines Verteilen der Lösung in Wasser erhält man eine wässrige Dispersion.

III. 20 Gew.-Teile der Verbindung des Beispiels 5 werden in einer Mischung gelöst, die aus 40 Gew.-Teilen Cyclohexanon, 30 Gew.-Teilen Isobutanol, 20 Gew.-Teilen des Anlagerungsproduktes von 40 Mol Ethylenoxid an 1 Mol Ricinusöl besteht. Durch Eingiessen und feines Verteilen der Lösung in Wasser erhält man eine wässrige Dispersion.

IV. 20 Gew.-Teile der Verbindung des Beispiels 10 werden in einer Mischung gelöst, die aus 25 Gew.-Teilen Cyclohexanol, 65 Gew.-Teilen einer Mineralölfraktion vom Siedepunkt 210 bis 280°C und 10 Gew.-Teilen des Anlagerungsproduktes von 40 Mol Ethylenoxid an 1 Mol Ricinusöl besteht. Durch Eingiessen und feines Verteilen der Lösung in Wasser erhält man eine wässrige Dispersion.

V. 80 Gew.-Teile der Verbindung des Beispiels 17 werden mit 3 Gew.-Teilen des Natriumsalzes der Diisobutylnaphthalin-alpha-sulfonsäure, 10 Gew.-Teilen des Natriumsalzes einer Ligninsulfonsäure aus einer Sulfitablauge und 7 Gew.-Teilen pulverförmigem Kieselsäuregel gut vermischt und in einer Hammermühle vermahlen. Durch feines Verteilen der Mischung in Wasser erhält man eine Spritzbrühe.

VI. 3 Gew.-Teile der Verbindung des Beispiels 24 werden mit 97 Gew.-Teilen feinteiligem Kaolin innig vermischt. Man erhält auf diese Weise ein Stäubemittel das 3 Gew.-% des Wirkstoffs enthält.

VII. 30 Gew.-Teile der Verbindung des Beispiels 18 werden mit einer Mischung aus 92 Gew.-Teilen pulverförmigem Kieselsäuregel und 8 Gew.-Teilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.

VIII. 40 Gew.-Teile der Verbindung des Beispiels 30 werden mit 10 Teilen Natriumsalz eines Phenolsulfonsäureharnstoff-formaldehyd-Kondensates, 2 Teilen Kieselgel und 48 Teilen Wasser innig vermischt. Man erhält eine stabile wässrige Dispersion. Durch Verdünnen mit Wasser erhält man eine wässrige Dispersion.

IX. 20 Teile der Verbindung des Beispiels 24 werden mit 2 Teilen Calciumsalz der Dodecylbenzolsulfonsäure, 8 Teilen Fettalkohol-polyglykolether, 2 Teilen Natriumsalz eines Phenolsulfonsäure-harnstoff-formaldehyd-Kondensats und 68 Teilen eines paraffinischen Mineralöls innig vermischt. Man erhält eine stabile ölige Dispersion.

Die erfindungsgemässen Mittel können in diesen Anwendungsformen auch zusammen mit anderen Wirkstoffen vorliegen, wie z.B. Herbiziden, Insektiziden, Wachstumsregulatoren und Fungiziden, oder auch mit Düngemitteln vermischt und ausgebracht werden. Beim Vermischen mit Fungiziden erhält man dabei in vielen Fällen eine Vergrösserung des fungiziden Wirkungsspektrums.

Die folgende Liste von Fungiziden, mit denen die erfindungsgemässen Verbindungen kombiniert werden können, soll die Kombinationsmöglichkeiten erläutern, nicht aber einschränken.

Fungizide, die mit den erfindungsgemässen Verbindungen kombiniert werden können, sind beispielsweise:

Schwefel,
Dithiocarbamate und deren Derivate, wie
Ferridimethyldithiocarbamat,
Zinkdimethyldithiocarbamat,
Manganthylenbisdithiocarbamat,
Mangan-Zink-ethylendiamin-bis-dithiocarbamat und Zinkethylenbisdithiocarbamat.
Tetramethylthiuramdisulfide,
Ammoniak-Komplex von Zink-(N,N-ethylen-bis-dithiocarbamat) und N,N'-Polyethylen-bis-(thiocarbamoyl)-disulfid.
Zink-(N,N'-propylen-bis-dithiocarbamat),
Ammoniak-Komplex von Zink-(N,N'-propylen-bis-dithiocarbamat) und N,N'-Polypropylen-bis-(thiocarbamoyl)-disulfid;
Nitroderivate, wie
Dinitro-(1-methylheptyl)-phenylcrotonat,
2-sec.-Butyl-4,6-dinitrophenyl-3,3-dimethylacrylat,
2-sec.-Butyl-4,6-dinitrophenyl-isopropylcarbonat;
heterocyclische Substanzen, wie
N-(1,1,2,2-Tetrachlorethylthio)-tetrahydrophthalimid,
N-Trichlormethylthio-tetrahydrophthalimid,
2-Heptadecyl-2-imidazolin-acetat
2,4-Dichlor-6-(o-chloranilino)-s-triazin
O,O-Diäthyl-phthalimidophonothioat
5-Amino-1-(bis-(dimethylamino)-phosphinyl)-3-phenyl-1,2,4-triazol)
2,3-Dicyano-1,4-Dithioaanthrachinon
5-Ethoxy-3-trichlormethyl-1,2,4-thiadiazol,
2-Thio-1,3-dithio-(4,5-b)-chinoxalin
1-(Butylcarbamoyl)-2-benzimidazol-carbaminsäuremethylester
2-Methoxycarbonylamino-benzimidazol,
2-Rhodanmethylthio-benzthiazol,
4-(2-Chlorphenylhydrazono)-3-methyl-5-isoxazolon
Pyridin-2-thio-1-oxid
8-Hydroxychinolin bzw. dessen Kupfersalz
2,3-Dihydro-5-carboxanilido-6-methyl-1,4-oxathiin-4,4-dioxid
2,3-Dihydro-5-carboxanilido-6-methyl-1,4-oxathiin
2-(Furyl-(2))-benzimidazol
Piperazin-1,4-diyl-bis-(1-(2,2,2-tri-chlorethyl'-formamid
2-(Thiazolyl-(4))-benzimidazol
5-Butyl-2-dimethylamino-4-hydroxy-6-methyl-pyrimidin
Bis-(p-chlorphenyl)-3-pyridinmethanol
1,2-Bis-(3-ethoxycarbonyl-2-thioureido)-benzol
1,2-Bis-(3-methoxycarbonyl-2-thioureido)-benzol
und weitere Substanzen, wie
Dodecylguanidinacetat,
3-(3-(3,5-dimethyl-2-oxycyclohexyl)-2-hydroxyethyl)-glutaramid
Hexachlorbenzol

N-Dichlorfluormethylthio-N',N'-dimethyl-N-phenyl-schwefelsäurediamid
2,5-Dimethyl-furan-3-carbonsäureanilid
2,5-Dimethyl-furan-3-carbonsäure-cyclohexylamid,
2-Cyan-N-(ethylaminocarbonyl)-2-(methoxyimino)-acetamid,
2-Methyl-benzoesäure-anilid
2-Iodbenzoesäure-anilid
1-(3,4-Dichloranilino)-1-formylamino-2,2,2-trichlorethan
2,6-Dimethyl-N-tridecyl-morpholin bzw. dessen Salze
2,6-Dimethyl-N-cyclododecyl-morpholin bzw. dessen Salze

DL-Methyl-N-(2,6-dimethyl-phenyl)-N-furoyl(2)-alaninat
DL-N-(2,6-Dimethyl-phenyl)-N-(2'-methoxyacetyl)-alanin-methylester
5-Nitro-isophthalsäure-di-isopropylester
1-(1',2',4'-Triazolyl-1')-1-(4'-chlorphenoxy)-3,3-dimethylbutan-2-on
1-(1',2',4'-Triazolyl-1')-1-(4'-chlorphenoxy)-3,3-dimethylbutan-2-ol
N-(2,6-Dimethylphenyl)-N-chloracetyl-D,L-2-aminobutyrolacton
N-(n-Propyl)-N-(2,4,6-trichlorphenoxyethyl)-N'-imidazolyl-harnstoff.

Die folgenden Versuche zeigen die fungizide Wirkung der neuen Substanzen. Als Vergleichssubstanzen dienten die bekannten Wirkstoffe (1-(2'-(2",4"-Dichlorphenyl)-2'-(2"-propenyloxy)-ethyl)-1H-imidazol) (A) sowie das 2,2-Dimethyl-4-(1,2,4-triazol- 1-yl)-5-phenyl- pentanon-(3) (B).

Versuch 1

Wirksamkeit gegen Weizenmehltau

Blätter von in Töpfen gewachsenen Weizenkeimlingen der Sorte «Jubilar» werden mit wässrigen Emulsionen aus 80% (Gew.-%) Wirkstoff und 20% Emulgiermittel (bezogen auf die Trockensubstanz) besprüht und nach dem Antrocknen des Spritzbelages mit Oidien (Sporen) des Weizenmehltaus (Erysiphe graminis var. tritici) bestäubt. Die Versuchspflanzen werden anschliessend im Gewächshaus bei Temperaturen zwischen 20 und 22°C und 75 bis 80% relativer Luftfeuchtigkeit aufgestellt. Nach 10 Tagen wird das Ausmass der Mehltauentwicklung ermittelt.

Bei diesem Versuch zeigten die Wirkstoffe 4, 5, 13, 16, 17, 18, 24, 30 eine bessere fungizide Wirkung als der Wirkstoff A.

Versuch 2

Wirksamkeit gegen Weizenbraunrost

Blätter von in Töpfen gewachsenen Weizensämlingen der Sorte «Caribo» werden mit Sporen des Braunrostes (Puccinia recondita) bestäubt. Danach werden die Töpfe für 24 Std. bei 20 bis 22°C in eine Kammer mit hoher Luftfeuchtigkeit (90 bis 95%) gestellt. Während dieser Zeit keimen die Sporen aus und die Keimschläuche dringen in das Blattgewebe ein. Die infizierten Pflanzen werden anschliessend mit einer 0,025%igen (Gew.-%) wässrigen Spritzbrühe, die 80% Wirkstoff und 20% Ligninsulfonat in der Trockensubstanz enthält, tropfnass gespritzt. Nach dem Antrocknen des Spritzbelages werden die Versuchspflanzen im Gewächshaus bei Temperaturen zwischen 20 und 22°C und 65 bis 70% relativer Luftfeuchte aufgestellt. Nach 8 Tagen wird das Ausmass der Rostpilzentwicklung auf den Blättern ermittelt.

Bei diesem Versuch zeigten die Wirkstoffe 5, 10, 17, 18 und 24 eine bessere fungizide Wirkung als die Wirkstoffe A und B.

Versuch 3

Wirksamkeit gegen Gurkenmehltau

Blätter von in Töpfen gewachsenen Gurkenkeimlingen werden mit wässrigen Emulsionen aus 80% Wirkstoff und 20% Emulgiermittel (Ligninsulfonat, bezogen auf die Trockensubstanz) besprüht und nach dem Antrocknen des Spritzbelages mit Oidien (Sporen) des Gurkenmehltaus (Erysiphe cichoracearum) bestäubt. Die Versuchspflanzen werden anschliessend im Gewächshaus bei Temperaturen zwischen 20 und 22°C und 75 bis 80% relativer Luftfeuchtigkeit aufgestellt. Nach 10 Tagen wird das Ausmass der Mehltaupilzentwicklung ermittelt.

In diesem Versuch zeigten die Wirkstoffe 1, 4, 5, 7, 8, 12, 13, 15, 16, 17, 18, 19, 20, 22, 24, 26, 28, 29, 30, 31, 32 und 33 eine gute fungizide Wirkung.

**Patentansprüche für die Vertragsstaaten: BE, CH, DE, FR, GB, IT, LI, NL, SE**

1. Azolylalkyl-2,3-dihydrobenzofuran der Formel

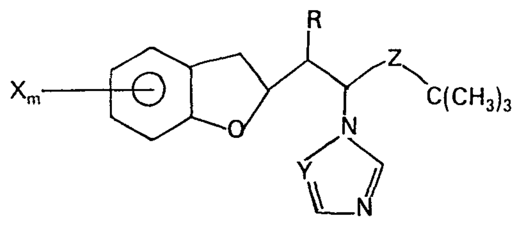

in der

R Wasserstoff oder Alkyl mit 1 bis 4 C-Atomen bedeutet,

X Wasserstoff, Fluor, Chlor, Brom, Jod, Nitro, Trifluormethyl oder Alkyl mit 1 bis 5 C-Atomen, Alkoxy mit 1 bis 3 C-Atomen oder Alkenyl mit 2 bis 3 C-Atomen, Phenyl oder Phenoxy bedeutet und

m eine ganze Zahl von 1 bis 4 bedeutet, wobei die einzelnen Gruppen X gleich oder verschieden sind, wenn m grösser als 1 ist,

Y N oder CH bedeutet und

Z eine -CO- oder $-CR^1OR^2$-Gruppe bedeutet, in welcher

$R^1$ Wasserstoff oder $C_1$–$C_4$-Alkyl bedeutet und

$R^2$ Wasserstoff, einen gegebenenfalls durch Chlor substituierten Alkylrest mit 1 bis 6 C-Atomen oder einen gegebenenfalls durch

Chlor substituierten Alkenylrest mit 3 bis 5 C-Atomen oder einen Alkinylrest mit 3 bis 4 C-Atomen oder Benzyl bedeutet, wobei der Benzylrest durch Fluor, Chlor, Brom, Nitro, Trifluormethyl und Alkyl mit 1 bis 4 C-Atomen substituiert sein kann, oder $R^2$ einen $-CO-R^3$-Rest bedeutet, wobei

$R^3$ einen gegebenenfalls durch Halogen, Alkoxy mit 1 bis 2 C-Atomen, Oxo (=O) oder Carboxyalkyl mit 2 bis 5 C-Atomen substituierten Alkylrest mit 1 bis 4 C-Atomen

bedeutet, sowie dessen Säureadditionssalze und Metallkomplexsalze.

2. Fungizid enthaltend ein Azolylalkyl-2,3-dihydrobenzofuran gemäss Anspruch 1.

3. Fungizid enthaltend einen festen oder flüssigen Trägerstoff und ein Azolylalkyl-2,3-dihydrobenzofuran gemäss Anspruch 1.

4. Verfahren zur Herstellung eines Fungizids, dadurch gekennzeichnet, dass man einen festen oder flüssigen Trägerstoff vermischt mit einem Azolylalkyl-2,3-dihydrobenzofuran gemäss Anspruch 1.

5. Verfahren zur Bekämpfung von Pilzen, dadurch gekennzeichnet, dass man die Pilze oder die vor Pilzbefall zu schützenden Gegenstände behandelt mit einem Azolylalkyl-2,3-dihydrobenzofuran gemäss Anspruch 1.

6. Verfahren zur Herstellung eines Azolylalkyl-2,3-dihydrobenzofurans gemäss Anspruch 1, dadurch gekennzeichnet, dass man 2,3-Dihydrobenzofuran-2-methylhalogenid der Formel

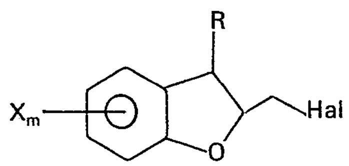

in der R, X und m die oben angegebenen Bedeutungen haben und Hal Chlor, Brom oder Jod bedeutet, mit einem Keton der Formel

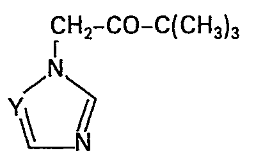

in der Y die angegebene Bedeutung hat oder mit dessen Alkalienolat, gegebenenfalls in Gegenwart eines Lösungs- oder Verdünnungsmittels, gegebenenfalls in Gegenwart einer starken anorganischen oder organischen Base und gegebenenfalls unter Zusatz eines Reaktionsbeschleunigers und/oder Phasentransferkatalysators bei Temperaturen zwischen 0 und 100°C, umsetzt und die so erhaltenen Verbindungen gemäss Anspruch 1, in denen Z eine CO-Gruppe bedeutet, gegebenenfalls durch Einwirkung von Wasserstoff in Gegenwart eines Hydrierungskatalysators oder durch Einwirkung eines komplexen Hydrids oder durch Einwirkung von Aluminiumisopropoxid und Isopropanol oder durch Einwirkung von Natriumdithionit oder elektrochemisch gegebenenfalls in Gegenwart eines Lösungs- oder Verdünnungsmittels bei Temperaturen zwischen 0 und 100°C zu sekundären Alkoholen gemäss Anspruch 1, in denen Z eine CHOH-Gruppe bedeutet, reduziert oder mit einer Grignardverbindung der Formel

$R^1-MgHal$

in der $R^1$ $C_1$–$C_4$-Alkyl bedeutet und in der Hal Chlor, Brom oder Jod bedeutet, gegebenenfalls in Gegenwart eines Lösungs- oder Verdünnungsmittels und gegebenenfalls in Gegenwart eines Magnesium- oder Tetraalkylammonium-halogenids bei Temperaturen zwischen 0 und 100°C zu tertiären Alkoholen gemäss Anspruch 1, in denen Z eine $R^1COH$-Gruppe bedeutet, umsetzt und die so entstandenen tertiären und sekundären Alkohole – falls gewünscht – mit einem Alkylierungsmittel der Formel

$R^2-Hal$

in der $R^2$ die im Anspruch 1 angegebene Bedeutung hat und Hal Chlor, Brom und Jod bedeutet oder mit einem Säurechlorid der Formel $R^3-COCl$ oder mit einem Säureanhydrid der Formel $(R^3-CO)_2O$ in denen $R^3$ die im Anspruch 1 angegebene Bedeutung hat, gegebenenfalls in Gegenwart eines Lösungs- oder Verdünnungsmittels und/oder einer anorganischen oder organischen Base und/oder eines Reaktionsbeschleunigers und/oder eines Phasentransferkatalysators bei Temperaturen zwischen 0 und 100°C umsetzt und die so erhaltenen Verbindungen gegebenenfalls anschliessend mit Säure in ihre Säureadditionssalze oder mit Metallsalzen in ihre Metallkomplexe überführt.

7. Azolylalkyl-2,3-dihydrobenzofuran, ausgewählt aus der Gruppe bestehend aus 1-(2,3-Dihydro-5,7-dichlorbenzofuran-2-yl)-2-(1,2,4-triazol-1-yl)-3-methoxy-4,4-dimethylpentan, 1-(2,3-Dihydro-5,7-dichlorbenzofuran-2-yl)-(1,2,4-triazol-1-yl)-3-hydroxy-4,4-dimethyl-pentan, 1-(2,3-Dihydro-3-methyl-5,7-dichlorbenzofuran-2-yl)-2-(1,2,4-triazol-1-yl)-4,4-dimethylpentan-3-on.

8. Fungizid enthaltend ein Azolylalkyl-2,3-dihydrobenzofuran, ausgewählt aus der Gruppe bestehend aus 1-(2,3-Dihydro-5,7-dichlorbenzofuran-2-yl)-2-(1,2,4-triazol-1-yl)-3-methoxy-4,4-dimethylpentan, 2-(2,3-Dihydro-5,7-dichlorbenzofuran-2-yl)-2-(1,2,4-triazol-1-yl)-3-hydroxy-4,4-dimethylpentan, 1-(2,3-Dihydro-3-methyl-5,7-dichlorbenzofuran-2-yl)-2-(1,2,4-triazol-1-yl)-4,4-dimethylpentan-3-on.

**Patentansprüche für den Vertragsstaat: AT**

1. Fungizid enthaltend ein Azolylalkyl-2,3-dihydrobenzofuran der Formel

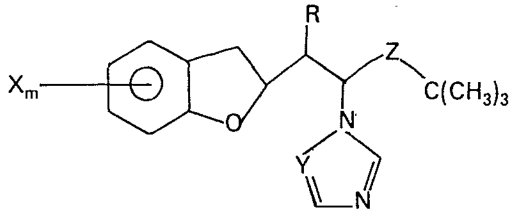

in der

R Wasserstoff oder Alkyl mit 1 bis 4 C-Atomen bedeutet,

X Wasserstoff, Fluor, Chlor, Brom, Jod, Nitro, Trifluormethyl oder Alkyl mit 1 bis 5 C-Atomen, Alkoxy mit 1 bis 3 C-Atomen oder Alkenyl mit 2 bis 3 C-Atomen, Phenyl oder Phenoxy bedeutet und

m eine ganze Zahl von 1 bis 4 bedeutet, wobei die einzelnen Gruppen X gleich oder verschieden sind, wenn m grösser als 1 ist,

Y N oder CH bedeutet und

Z eine -CO- oder $-CR^1OR^2$-Gruppe bedeutet, in welcher

$R^1$ Wasserstoff oder $C_1$–$C_4$-Alkyl bedeutet und

$R^2$ Wasserstoff, einen gegebenenfalls durch Chlor substituierten Alkylrest mit 1 bis 6 C-Atomen oder einen gegebenenfalls durch Chlor substituierten Alkenylrest mit 3 bis 5 C-Atomen oder einen Alkinylrest mit 3 bis 4 C-Atomen oder Benzyl bedeutet, wobei der Benzylrest durch Fluor, Chlor, Brom, Nitro, Trifluormethyl und Alkyl mit 1 bis 4 C-Atomen substituiert sein kann, oder $R^2$ einen $-CO-R^3$-Rest bedeutet, wobei

$R^3$ einen gegebenenfalls durch Halogen, Alkoxy mit 1 bis 2 C-Atomen, Oxo (=O) oder Carboxyalkyl mit 2 bis 5 C-Atomen substituierten Alkylrest mit 1 bis 4 C-Atomen bedeutet,

sowie dessen Säureadditionssalze und Metallkomplexsalze.

2. Fungizid enthaltend einen festen oder flüssigen Trägerstoff und ein Azolylalkyl-2,3-dihydrobenzofuran gemäss Anspruch 1.

3. Verfahren zur Herstellung eines Fungizids, dadurch gekennzeichnet, dass man einen festen oder flüssigen Trägerstoff vermischt mit einem Azolylalkyl-2,3-dihydrobenzofuran gemäss Anspruch 1.

4. Verfahren zur Bekämpfung von Pilzen, dadurch gekennzeichnet, dass man die Pilze oder die vor Pilzbefall zu schützenden Gegenstände behandelt mit einem Azolylalkyl-2,3-dihydrobenzofuran gemäss Anspruch 1.

5. Verfahren zur Herstellung eines Azolylalkyl-2,3-dihydrobenzofurans gemäss Anspruch 1, dadurch gekennzeichnet, dass man 2,3-Dihydrobenzofuran-2-methylhalogenid der Formel

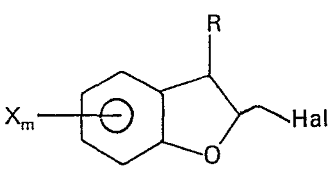

in der R, X und m die oben angegebene Bedeutungen haben und Hal Chlor, Brom oder Jod bedeutet, mit einem Keton der Formel

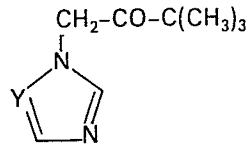

in der Y die angegebene Bedeutung hat oder mit dessen Alkalienolat, gegebenenfalls in Gegenwart eines Lösungs- oder Verdünnungsmittels, gegebenenfalls in Gegenwart einer starken anorganischen oder organischen Base und gegebenenfalls unter Zusatz eines Reaktionsbeschleunigers und/oder Phasentransferkatalysators bei Temperaturen zwischen 0 und 100°C, umsetzt und die so erhaltenen Verbindungen gemäss Anspruch 1, in denen Z eine CO-Gruppe bedeutet, gegebenenfalls durch Einwirkung von Wasserstoff in Gegenwart eines Hydrierungskatalysators oder durch Einwirkung eines komplexen Hydrids oder durch Einwirkung von Aluminiumisopropoxid und Isopropanol oder durch Einwirkung von Natriumdithionit oder elektrochemisch gegebenenfalls in Gegenwart eines Lösungs- oder Verdünnungsmittels bei Temperaturen zwischen 0 und 100°C zu sekundären Alkoholen gemäss Anspruch 1, in denen Z eine CHOH-Gruppe bedeutet, reduziert oder mit einer Grignardverbindung der Formel

$R^1-MgHal$

in der $R^1$ $C_1$–$C_4$-Alkyl bedeutet und in der Hal Chlor, Brom oder Jod bedeutet, gegebenenfalls in Gegenwart eines Lösungs- oder Verdünnungsmittels und gegebenenfalls in Gegenwart eines Magnesium- oder Tetraalkylammonium-halogenids bei Temperaturen zwischen 0 und 100°C zu tertiären Alkoholen gemäss Anspruch 1, in denen Z eine $R^1COH$-Gruppe bedeutet, umsetzt und die so entstandenen tertiären und sekundären Alkohole – falls gewünscht – mit einem Alkylierungsmittel der Formel

$R^2-Hal$

in der $R^2$ die im Anspruch 1 angegebene Bedeutung hat und Hal Chlor, Brom und Jod bedeutet oder mit einem Säurechlorid der Formel $R^3-COCl$ oder mit einem Säureanhydrid der Formel $(R^3-CO)_2O$ in denen $R^3$ die im Anspruch 1 angegebene Bedeutung hat, gegebenenfalls in Gegenwart eines Lösungs- oder Verdünnungsmittels und/oder einer anorganischen oder organischen Base und/oder eines Reaktionsbeschleunigers und/oder eines Phasentransferkatalysators bei Temperaturen zwischen 0 und 100°C umsetzt und die so erhaltenen Verbindungen gemäss Anspruch 1 gegebenenfalls anschliessend mit Säure in ihre Säureadditionssalze oder mit Metallsalzen in ihre Metallkomplexe überführt.

6. Fungizid, enthaltend ein Azolylalkyl-2,3-dihydrobenzofuran, ausgewählt aus der Gruppe bestehend aus 1-(2,3-Dihydro-5,7-dichlorbenzofuran-2-yl-2-(1,2,4-triazol-1-yl)-3-methoxy-4-dimethylpentan,1-(2,3-Dihydro-5,7-dichlorbenzofuran-2-yl)-2-(1,2,4-triazol-1-yl)-3-hydroxy-4,4-dimethylpentan, 1-(2,3-Dihydro-3-methyl-5,7-dichlorbenzofuran-2-yl)-2-(1,2,4-triazol-1-yl)-4,4-dimethylpentan-3-on.

**Claims for the Contracting States: BE, CH, DE, FR, GB, IT, LI, LU, NL, SE**

1. An azolylalkyl-2,3-dihydrobenzofuran of the formula

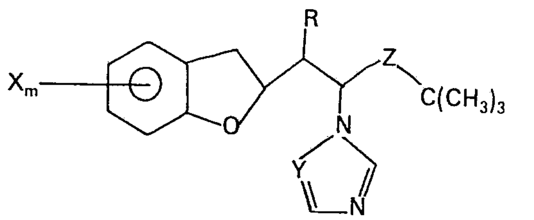

where
R is hydrogen or alkyl of 1 to 4 carbon atoms,
X is hydrogen, fluorine, chlorine, bromine, iodine, nitro, trifluormethyl, alkyl of 1 to carbon atoms, alkoxy of 1 to 3 carbon atoms, alkenyl of 2 or 3 carbon atoms, phenyl or phenoxy, and
m is an integer from 1 to 4, and the individual groups X are identical or different if m is greater than 1,
Y is N or CH and
Z is $-CO-$ or $-CR^1OR^2-$, where
$R^1$ is hydrogen or alkyl of 1 to 4 carbon atoms, and
$R^2$ is hydrogen, or is alkyl of 1 to 6 carbon atoms which is unsubstituted or substituted by chlorine, or is alkenyl of 3 to 5 carbon atoms which is unsubstituted or substituted by chlorine, or is alkynyl of 3 or 4 carbon atoms, or is benzyl, which may be substituted by fluorine, chlorine, bromine, nitro, trifluoromethyl or alkyl of 1 to 4 carbon atoms, or is $-CO-R^3-$, where
$R^3$ is alkyl of 1 to 4 carbon atoms which is unsubstituted or substituted by halogen, alkoxy of 1 to 2 carbon atoms, oxo (=O) or carboxyalkyl of 2 to 5 carbon atoms,

or an acid addition salt or metal salt complex thereof.

2. A fungicide containing an azolylalkyl-2,3-dihydrobenzofuran as claimed in claim 1.

3. A fungicide containing a solid or liquid carrier and an azolylalkyl-2,3-dihydrobenzofuran as claimed in claim 1.

4. A process for manufacturing a fungicide, wherein a solid or liquid carrier is mixed with an azolylalkyl-2,3-dihydrobenzofuran as claimed in claim 1.

5. A process for combating fungi, wherein the fungi or the objects to be protected against fungus attack are treated with an azolylalkyl-2,3-dihydrobenzofuran as claimed in claim 1.

6. A process for the manufacture of an azolylalkyl-2,3-dihydrobenzofuran as claimed in claim 1, wherein a 2,3-dihydrobenzofuran-2-yl-methyl halide of the formula

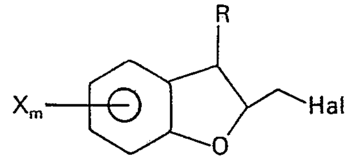

where R, X and m have the above meanings, and Hal is chlorine, bromine or iodine, is reacted with a ketone of the formula

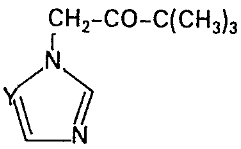

where Y has the above meaning, or with an alkali metal enolate thereof, in the presence or absence of a solvent or diluent, in the presence or absence of a strong inorganic or organic base, and with or without the addition of a reaction accelerator and/or phase transfer catalyst, at from 0 to 100°C, and the compounds according to claim 1 thus obtained (Z being $-CO-$) are, if desired, reduced in the presence of a solvent or diluent, at from 0 to 100°C, with hydrogen in the presence of a hydrogenation catalyst, or with a complex hydride, or with aluminum isopropoxide and isopropanol, or with sodium dithionite or electrochemically, to give secondary alcohols as claimed in claim 1, in which Z is the group 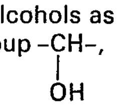,

or the compounds as claimed in claim 1 (Z being $-CO-$) are reacted with a Grignard compound of the formula

$R^1MgHal$,

where $R^1$ is alkyl of 1 to 4 carbon atoms, and Hal is chlorine, bromine or iodine, in the presence or absence of a solvent or diluent and in the presence or absence of a magnesium or tetraalkylammonium halide, at from 0 to 100°C, to give tertiary alcohols as claimed in claim 1, where Z is the group

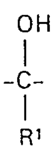

and the tertiary and secondary alcohols thus obtained are, if desired, reacted with an alkylating agent of the formula

$R^2-Hal$,

where $R^2$ has the meanings given in claim 1, and Hal is chlorine, bromine or iodine, or with an acid chloride of the formula $R^3$–COCl or with an acid anhydride of the formula $(r^3–CO)_2O$, $R^3$ having the meanings given in claim 1, in the presence or absence of a solvent or diluent and/or an inorganic or organic base and/or a reaction accelerator and/or a phase transfer catalyst, at from 0 to 100°C, and the compounds thus obtained are, if desired, subsequently converted with acid into their acid addition salts or with metal salts into their metal salt complexes.

7. An azolylalkyl-2,3-dihydrobenzofuran selected from the group consisting of 1-(2,3-dihydro-5,7-dichlorobenzofuran-2-yl)-2-(1,2,4-triazol-1-yl)-3-methoxy-4,4-dimethylpentane, 1-(2,3-dihydro-5,7-dichlorobenzofuran-2-yl)-2-(1,2,4-triazol-1-yl)-3-hydroxy-4,4-dimethylpentane, and 1-(2,3-dihydro-3-methyl-5,7-dichlorobenzofuran-2-yl)-2-(1,2,4-triazol-1-yl)-4,4-dimethylpentan-3-one.

8. A fungicide containing an azolylalkyl-2,3-dihydrobenzofuran selected from the group consisting of 1-(2,3-dihydro-5,7-dichlorobenzofuran-2-yl)-2-(1,2,4-triazol-1-yl)-3-methoxy-4,4-dimethylpentane, 1-(2,3-dihydro-5,7-dichlorobenzofuran-2-yl)-2-(1,2,4-triazol-1-yl)-3-hydroxy-4,4-dimethylpentane, and 1-(2,3-dihydro-3-methyl-5,7-dichlorobenzofuran-2-yl)-2-(1,2,4-triazol-1-yl)-4,4-dimethylpentan-3-one.

**Claims for the Contracting State: AT**

1. A fungicide containing an azolylalkyl-2,3-dihydrobenzofuran of the formula

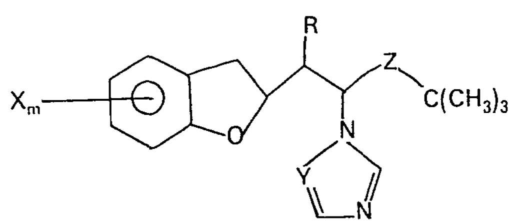

where

R is hydrogen or alkyl of 1 to 4 carbon atoms,

X is hydrogen, fluorine, chlorine, bromine, iodine, nitro, trifluoromethyl, alkyl of 1 to 5 carbon atoms, alkoxy of 1 to 3 carbon atoms, alkenyl of 2 or 3 carbon atoms, phenyl or phenoxy, and

m is an integer from 1 to 4, and the individual groups X are identical or different if m is greater than 1,

Y is N or CH and Z is –CO– or $–CR^1OR^2–$, where

$R^1$ is hydrogen or alkyl of 1 to 4 carbon atoms, and

$R^2$ is hydrogen, or is alkyl of 1 to 6 carbon atoms which is unsubstituted or substituted by chlorine, or is alkenyl of 3 to 5 carbon atoms which is unsubstituted or substituted by chlorine, or is alkynyl of 3 or 4 carbon atoms, or is benzyl, which may be substituted by fluorine, chlorine, bromine, nitro, trifluoromethyl or alkyl of 1 to 4 carbon atoms, or is $–CO–R^3–$, where

$R^3$ is alkyl of 1 to 4 carbon atoms which is unsubstituted or substituted by halogen, alkoxy of 1 to 2 carbon atoms, oxo (=O) or carboxyalkyl of 2 to 5 carbon atoms,

or an acid addition salt or metal salt complex thereof.

2. A fungicide containing a solid or liquid carrier and an azolylalkyl-2,3-dihydrobenzofuran as defined in claim 1.

3. A process for manufacturing a fungicide, wherein a solid or liquid carrier is mixed with an azolylalkyl-2,3-dihydrobenzofuran as defined in claim 1.

4. A process for combating fungi, wherein the fungi or the objects to be protected against fungus attack are treated with an azolylalkyl-2,3-dihydrobenzofuran as defined in claim 1.

5. A process for the manufacture of an azolylalkyl-2,3-dihydrobenzofuran as defined in claim 1, wherein a 2,3-dihydrobenzofuran-2-yl-methyl halide of the formula

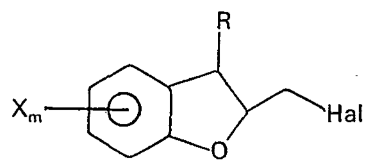

where R, X and m have the above meanings, and Hal is chlorine, bromine or iodine, is reacted with a ketone of the formula

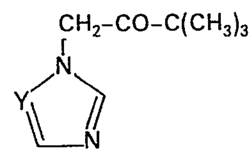

where Y has the above meaning, or with an alkali metal enolate thereof, in the presence or absence of a solvent or diluent, in the presence or absence of a strong inorganic or organic base, and with or without the addition of a reaction accelerator and/or phase transfer catalyst, at from 0 to 100°C, and the compounds according to claim 1 thus obtained (Z being –CO–) are, if desired, reduced in the presence of a solvent or diluent, at from 0 to 100°C, with hydrogen in the presence of a hydrogenation catalyst, or with a complex hydride, or with aluminum isopropoxide and isopropanol, or with sodium dithionite or electrochemically, to give secondary alcohols as claimed in claim 1, in which Z is the group –CH–,

OH,

or the compounds as claimed in claim 1 (Z being –CO–) are reacted with a Grignard compound of the formula

$R^1MgHal$,

where $R^1$ is alkyl of 1 to 4 carbon atoms, and Hal is chlorine, bromine or iodine, in the presence or

absence of a solvent or diluent and in the presence or absence of a magnesium or tetraalkylammonium halide, at from 0 to 100°C, to give tertiary alcohols as claimed in claim 1, where Z is the group

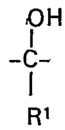

and the tertiary and secondary alcohols thus obtained are, if desired, reacted with an alkylating agent of the formula

$R^2$-Hal,

where $R^2$ has the meanings given in claim 1, and Hal is chlorine, bromine or iodine, or with an acid chloride of the formula $R^3$-COCl or with an acid anhydride of the formula $(R^3-CO)_2O$, $R^3$ having the meanings given in claim 1, in the presence or absence of a solvent or diluent and/or an inorganic or organic base and /or a reaction accelerator and/or a phase transfer catalyst, at from 0 to 100°C, and the compounds thus obtained are, if desired, subsequently converted with acid into their acid addition salts or with metal salts into their metal salt complexes.

6. A fungicide containing an azolylalkyl-2,3-dihydrobenzofuran selected from the group consisting of 1-(2,3-dihydro-5,7-dichlorobenzofuran-2-yl)-2-(1,2,4-triazol-1-yl)-3-methoxy-4,4-dimethylpentane, 1-(2,3-dihydro-5,7-dichlorobenzofuran-2-yl)-2-(1,2,4-triazol-1-yl)-3-hydroxy-4,4-dimethylpentane, and 1-(2,3-dihydro-3-methyl-5,7-dichlorobenzofuran-2-yl)-2-(1,2,4-triazol-1-yl)-4,4-dimethylpentan-3-one.

**Revendications pour les Etats contractants: BE, CH, DE, FR, GB, IT, LI, LU, NL, SE**

1. Azolylalkyl-2,3-dihydrobenzofurane de formule

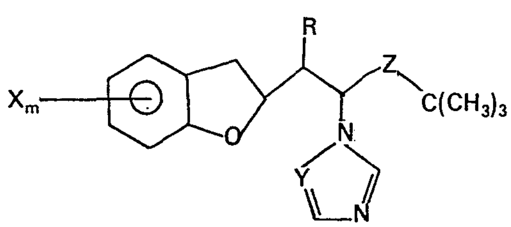

dans laquelle

R représente hydrogène ou alkyle à 1 à 4 atomes C,

X, hydrogène, fluor, chlore, brome, iode, nitro, trifluorométhyle ou alkyle à 1 à 5 atomes C, alcoxy à 1 à 3 atomes C ou alcényle à 2 à 3 atomes C, phényle ou phénoxy, et

m, représente un nombre entier de 1 à 4, les différents groupes X étant identiques ou différents, si m est supérieur à 1,

Y représente N ou CH et

Z un groupe -CO- ou $-CR^1OR^2$, où

$R^1$ représente hydrogène ou alkyle en $C_1$-$C_4$ et

$R^2$ hydrogène, un reste alkyle à 1 à 6 atomes C, éventuellement substitué par chlore, ou un reste alcényle à 3 à 5 atomes C, éventuellement substitué par chlore, ou un reste alcinyle à 3 à 4 atomes C ou benzyle, le reste benzyle pouvant être substitué par fluor, chlore, brome, nitro, trifluorométhyle et alkyle à 1 à 4 atomes C, ou $R^2$ représente un reste $-CO-R^3$,

$R^3$ représentant un reste alkyle à 1 à 4 atomes C, éventuellement substitué par halogène, alcoxy à 1 à 2 atomes C, oxo (= O) ou carboxyalkyle à 2 à 5 atomes C

ainsi que ses sels d'addition d'acide et ses sels complexes métalliques.

2. Fongicide contenant un azolylalkyl-2,3-dihydrobenzofurane selon la revendication 1.

3. Fongicide contenant un support solide ou liquide et un azolylalkyl-2,3-dihydrobenzofurane selon la revendication 1.

4. Procédé de préparation d'un fongicide, caractérisé par le fait qu'on melange un support solide ou liquide avec un azolylalkyl-2,3-dihydrobenzofurane selon la revendication 1.

5. Procédé de lutte contre les champignons, caractérisé par le fait qu'on traite les champignons, ou les objets à protéger contre l'attaque des champignons, avec un azolylalkyl-2,3-dihydrobenzofurane selon la revendication 1.

6. Procédé de préparation d'un azolylalkyl-2,3-dihydrobenzofurane selon la revendication 1, caractérisé par le fait qu'on fait réagir le 2,3-dihydrobenzofuran-2-méthylhalogénure de formule

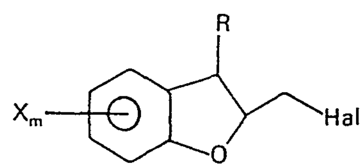

dans laquelle R, X et m ont les significations données plus haut, et Hal représente chlore, brome ou iode, avec une cétone de formule

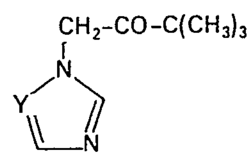

dans laquelle Y a la signification donnée, ou avec son alcaliénolate, éventuellement en présence d'un solvant ou diluant, éventuellement en présence d'une base forte inorganique ou organique, et éventuellement avec addition d'un accélérateur de réaction et/ou catalyseur de transfert de phases, à des températures comprises entre 0 et 100°C, et on réduit les composés ainsi obtenus selon la revendication 1, dans lesquels Z représente un groupe CO, en alcools secondaires selon la revendication 1, dans lesquels Z représente un groupe CHOH, éventuellement par action d'hydrogène en présence d'un catalyseur

d'hydrogénation ou par action d'un hydrure complexe ou par action d'isopropoxyde d'aluminium et d'isopropanol ou par action de dithionite de sodium ou électrochimiquement, éventuellement en présence d'un solvant ou diluant, à des températures entre 0 et 100°C, ou bien on les fait réagir avec un composé de Grignard de formule

$R^1$ - MgHal

dans laquelle $R^1$ représente alkyle en $C_1$–$C_4$ et où Hal représente chlore, brome ou iode, éventuellement en présence d'un solvant ou diluant et éventuellement en présence d'un halogénure de magnésium ou tétralkylammonium, à des températures comprises entre 0 et 100°C, pour obtenir des alcools tertiaires selon la revendication 1, dans lesquels Z représente un groupe $R^1COH$ et on fait réagir les alcools tertiaires et secondaires ainsi obtenus – si on le souhaite – avec un agent d'alkylation de formule

$R^2$ - Hal

dans laquelle $R^2$ a la signification donnée dans la revendication 1 et Hal représente chlore, brome et iode, ou avec un chlorure d'acide de formule $R^3$–COCl, ou avec un anhydride d'acide de formule $(R^3\text{–}CO)_2O$, dans laquelle $R^3$ a la signification donnée dans la revendication 1, éventuellement en présence d'un solvant ou diluant et/ou d'une base inorganique ou organique et/ou d'un accélérateur de réaction et/ou d'un catalyseur de transfert de phase, à des températures comprises entre 0 et 100°C, et l'on transforme les composés ainsi obtenus, éventuellement, ensuite, avec des acides, en leurs sels d'addition d'acide, ou, avec des sels métalliques, en leurs complexes métalliques.

7. Azolylalkyl-2,3-dihydrobenzofurane, choisi dans la groupe constitué par 1-(2,3-dihydro-5,7-dichlorobenzofuran-2-yl)-2-(1,2,4-triazol-1-yl)-3-méthoxy-4,4-diméthylpentane, 1-(2,3-dihydro-5,7-dichlorobenzofuran-2-yl)-2-(1,2,4-triazol-1-yl)-3-hydroxy-4,4-diméthyl-pentane, 1-(2,3-dihydro-3-méthyl-5,7-dichlorobenzofuran-2-yl)-2-(1,2,4-triazol-1-yl)-4,4-diméthylpentan-3-one.

8. Fongicide contenant un azolylalkyl-2,3-dihydrobenzofurane, choisi dans le groupe constitué par 1-(2,3-dihydro-5,7-dichlorobenzofuran-2-yl)-2-(1,2,4-triazol-1-yl)-3-méthoxy-4,4-diméthylpentane, 1-(2,3-dihydro-5,7-dichlorobenzofuran-2-yl)-2-(1,2,4-triazol-1-yl)-3-hydroxy-4,4-diméthylpentane, 1-(2,3-dihydro-3-méthyl)-5,7-dichlorobenzofuran-2-yl)-2-(1,2,4-trizol-1-yl)-4,4-diméthylpentane-3-one.

**Revendications pour l'Etat contractant: AT**

1. Fongicide contenant un azolylalkyl-2,3-dichlorobenzofurane de formule

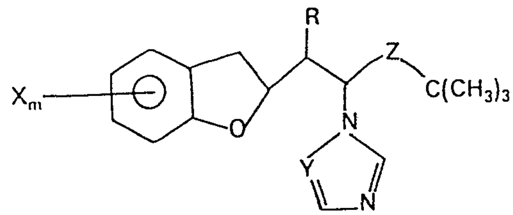

dans laquelle

- R représente hydrogène ou alkyle à 1 à 4 atomes C,
- X, hydrogène, fluor, chlore, brome, iode, nitro, trifluorométhyle ou alkyle à 1 à 5 atomes, C, alcocy, à 1 à 3 atomes C ou alcényle à 2 à 3 atomes C, phényle ou phénoxy, et
- m, représente un nombre entier de 1 à 4, les différents groupes X étant identiques ou différents, si m est supérieur à 1,
- Y représente N ou CH et
- Z un groupe -CO- ou -$CR^1OR^2$, où
- $R^1$ représente hydrogène ou alkyle en $C_1$–$C_4$ et
- $R^2$ hydrogène, un reste alkyle à 1 à 6 atomes C, éventuellement substitué par chlore, ou un reste alcényle à 3 à 5 atomes C, éventuellement substitué par chlore, ou un reste alcinyle à 3 à 4 atomes C ou benzyle, le reste benzyle pouvant être substitué par fluor, chlore, brome, nitro, trifluorméthyle et alkyle à 1 à 4 atomes C, ou $R^2$ représente un rest -CO-$R^3$
- $R^3$ représentant un reste alkyle à 1 à 4 atomes C, éventuellement substitué par halogène, alcoxy à 1 à 2 atomes C, oxo (= O) ou carboxyalkyle à 2 à 5 atomes C

ainsi que ses sels d'addition d'acide et ses sels complexes métalliques.

2. Fongicide contenant un support solide ou liquide et un azolylalkyl-2,3-dihydrobenzofurane selon la revendication 1.

3. Procédé de préparation d'un fongicide, caractérisé par le fait qu'on mélange un support solide ou liquide avec un azolylalkyl-2,3-dihydrobenzofurane selon la revendication 1.

4. Procédé de lutte contre les champignons, caractérisé par le fait qu'on traite les champignons ou les objects à protéger contre l'attaque des champignons avec un azolylalkyl-2,3-dihydrobenzofurane selon la revendication 1.

5. Procédé de préparation d'un azolylalkyl-2,3-dihydrobenzofurane selon la revendication 1, caractérisé par le fait qu'on fait réagir le 2,3-dihydrobenzofuran-2-méthyl-halogénure de formule

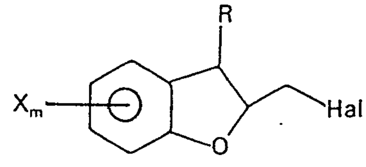

dans laquelle R, X et m ont les significations données plus haut, et Hal représente chlore, brome ou iode, avec une cétone de formule

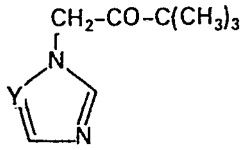

dans laquelle Y a la signification donnée ou avec son alcaliénolate, éventuellement en présence d'un solvant ou diluant, éventuellement en présence d'une base forte inorganique ou organique, et éventuellement avec addition d'un accélérateur de réaction et/ou catalyseur de transfert de phases, à des températures comprises entre 0 et 100°C, et ou réduit les composés ainsi obtenus selon la revendication 1, dans lesquels Z représente un groupe CO, en alcools secondaires selon la revendication 1, dans lesquels Z représente un groupe CHOH, éventuellement par action d'hydrogène en présence d'un catalyseur d'hydrogénation ou par action d'un hydrure complexe ou par action d'isopropoxyde d'aluminium et d'isopropanol ou par action de dithionite de sodium ou électrochimiquement, éventuellement en présence d'un solvant ou diluant, à des températures entre 0 et 100°C, ou bien on les fait réagir avec un composé de Grignard de formule

$R^1-MgHal$

dans laquelle $R^1$ représente alkyle en $C_1$–$C_4$ et où Hal représente chlore, brome ou iode, éventuellement en présence d'un solvant ou diluant et éventuellement en présence d'un halogénure de magnésium ou tétralkylammonium, à des températures comprises entre 0 et 100°C, pour obtenir des alcools tertiaires selon la revendication 1, dans lesquels Z représente un groupe $R^1COH$ et on fait réagir les alcools tertiaires et secondaires ainsi obtenus - si on le souhaite - avec un agent d'alkylation de formule

$R^2-Hal$

dans laquelle $R^2$ a la signification donnée dans la revendication 1 et Hal représente chlore, brome, et iode, ou avec un chlorure d'acide de formule $R^3$–COCl, ou avec un anhydride d'acide de formule $(R^3–CO)_2O$, dans laquelle $R^3$ a la signification donnée dans la revendication 1, éventuellement en présence d'un solvant ou diluant et/ou d'une base inorganique ou organique et/ou d'un accélérateur de réaction et/ou d'un catalyseur de transfert de phase, à des températures comprises entre 0 et 100°C, et l'on transforme les composés ainsi obtenus, éventuellement, ensuite, avec des acides, en leurs sels d'addition d'acide, ou, avec sels métalliques, en leurs complexes métalliques.

6. Fongicide contenant un azolylalkyl-2,3-dihydrobenzofurane, choisi dans le groupe constitué par 1-(2,3-dihydro-5,7-dichlorobenzofuran-2-yl)-2-(1,2,4-triazol-1-yl)-3-méthoxy-4,4-diméthylpentane, 1-(2,3-dihydro-5,7dichlorobenzofuran-2-yl)-2-(1,2,4-triazol-1-yl)-3-hydroxy-4,4-diméthylpentane, 1-(2,3-dihydro-3-méthyl-5,7-dichlorobenzofuran-2-yl)-2-(1,2,4-triazol-1-yl)-4,4-diméthylpentane-3-one.